# EUROPEAN PATENT APPLICATION

(11) **EP 4 537 833 A1**
(43) Date of publication of application: **16.04.2025**
(21) Application number: 22948057.9
(22) Date of filing: 06.12.2022
(51) Int. Cl.: A61K 31/7105, A61K 31/7088, A61K 48/00, A61P 1/16, A61P 11/00, A61P 13/12, C12N 15/113

(54) **PHARMACEUTICAL COMPOSITION FOR FIBROSIS TREATMENT**

(30) Priority: 20.06.2022 JP 2022098654
(71) Applicant: Kyorin Pharmaceutical Co., Ltd., Tokyo 100-0004 (JP)
(72) Inventor: ITO Minoru, Shimotsuga-gun, Tochigi 329-0114 (JP); MATOBA Toshihiro, Shimotsuga-gun, Tochigi 329-0114 (JP); HORIMOTO Satoshi, Shimotsuga-gun, Tochigi 329-0114 (JP); NAKAMURA Kazutaka, Shimotsuga-gun, Tochigi 329-0114 (JP); TSUTSUI Yusuke, Shimotsuga-gun, Tochigi 329-0114 (JP); OKADA Kazuyuki, Shimotsuga-gun, Tochigi 329-0114 (JP)
(74) Representative: Ricker, Mathias
(86) International application number: PCT/JP2022/044980
(87) International publication number: WO 2023/248498

(57) **Abstract**

Provided is a fibrosis treatment agent including, as an active ingredient, a nucleic acid that inhibits the expression of the NDR2 gene.

## Description

### Technical Field

The present invention relates to a pharmaceutical composition for treating fibrosis.

### Background Art

Fibrosis is a disease in which organ function is impaired with excessive accumulation of collagen to lead to irreversible progression, and for example, skin, lung, liver, pancreas, kidney, bone marrow, and the like have been known as the onset organs. Idiopathic pulmonary fibrosis (IPF), which is a type of fibrosis in the lung, is designated as an intractable disease in Japan because, although the disease prevalence is not high as represented by about twenty patients per one hundred thousand population, the post-treatment prognosis is undesirable as represented by the average survival period of 2.5 to 5 years after confirmation of diagnosis.

As therapeutic drugs for IPF, pirfenidon and nintedanib were approved by the Japanese Ministry of Health, Labour and Welfare and have been launched to date. Although both drugs show suppression of decline in vital capacity and prolongation action on progression-free survival and slow down progression of pathological condition, these cannot be deemed to be sufficiently satisfactory as therapeutic efficacies, and development of a therapeutic drug based on a new mechanism has been demanded.

Meanwhile, "NDR2 protein", which is a target of the present invention, is a serine/threonine kinase that is one of subtypes of Nuclear Dbf2-related kinases (NDR kinases) belonging to AGC family. There are two subtypes of NDR kinases: NDR1 (also known as STK38) and NDR2 (also known as STK38L). NDR kinases are widely present in cells throughout the body and are known to be involved in the morphogenesis of cells and tissues. On the other hand, it has been reported that no severe toxicity findings are observed in systemic knockout mice, particularly with respect to NDR2 (Non Patent Literature 1). To date, no association between NDR2 and fibrosis has been reported.

### Citation List

### Patent Literature

Patent Literature 1: International Publication No. 04/048576

### Non Patent Literature

Non Patent Literature 1: Debora Schmitz-Rohmer, et al., "PLOS ONE", 2015, vol. 10, e0136566

### Summary of Invention

### Technical Problem

An object of the present invention is to provide a novel therapeutic agent for fibrosis.

### Solution to Problem

The present inventors have conducted studies focusing on the fact that the addition of TGF-B1, which is a profibrotic factor, increases the expression level of the NDR2 gene in LL29 cells, which are human IPF pulmonary fibroblasts, and as a result, have found that the secretion of collagen, which is the main component of fibrosis, is suppressed by suppression of the expression of the NDR2 gene, thereby completing the present invention.

That is, the present invention is as follows.
[1] A therapeutic agent for fibrosis, comprising a nucleic acid suppressing expression of NDR2 gene as an active ingredient.
[2] The therapeutic agent according to [1], wherein the fibrosis is pulmonary fibrosis, hepatic fibrosis, or kidney fibrosis.
[3] The therapeutic agent according to [1] or [2], wherein the nucleic acid suppressing the expression of the NDR2 gene is an antisense nucleic acid.
[4] The therapeutic agent according to [1] or [2], wherein the nucleic acid suppressing the expression of the NDR2 gene is an siRNA.
[5] A method for treating fibrosis, comprising administering a nucleic acid suppressing expression of NDR2 gene to a subject.
[6] A nucleic acid suppressing expression of NDR2 gene for use in the treatment of fibrosis.
[7] Use of a nucleic acid suppressing expression of NDR2 gene in the manufacture of a therapeutic agent for fibrosis.
[8] The method, the nucleic acid, or the use according to [5] to [7], wherein the fibrosis is pulmonary fibrosis, hepatic fibrosis, or kidney fibrosis.
[9] The method, the nucleic acid, or the use according to [5] to [8], wherein the nucleic acid suppressing the expression of the NDR2 gene is an antisense nucleic acid.
[10] The method, the nucleic acid, or the use according to [5] to [8], wherein the nucleic acid suppressing the expression of the NDR2 gene is an siRNA.
[11] The therapeutic agent, the method, the nucleic acid, or the use according to [3] or [9], wherein the number of nucleotides contained in the antisense nucleic acid suppressing the expression of the NDR2 gene is 10- to 40-nt.
[12] The therapeutic agent, the method, the nucleic acid, or the use according to [3] or [9], wherein the number of nucleotides contained in the antisense nucleic acid suppressing the expression of the NDR2 gene is 12- to 21-nt.
[13] The therapeutic agent, the method, the nucleic acid, or the use according to [3] or [9], wherein an artificial nucleic acid is introduced into both ends of a sequence of the antisense nucleic acid suppressing the expression of the NDR2 gene.
[14] The therapeutic agent, the method, the nucleic acid, or the use according to [3] or [9], wherein the number of nucleotides contained in the antisense nucleic acid suppressing the expression of the NDR2 gene is 12- to 21-nt, and an artificial nucleic acid is introduced into both ends of a sequence.
[15] The therapeutic agent for fibrosis according to [1] or [2], wherein the nucleic acid suppressing the expression of the NDR2 gene is a nucleic acid comprising a guide strand and a passenger strand, the number of nucleotides contained in each of the guide strand and the passenger strand is 15- to 50-nt, and the guide strand comprises a sequence suppressing expression which is complementary to the NDR2 gene.
[16] The therapeutic agent according to [15], wherein the guide strand and/or the passenger strand is a nucleic acid molecule having an overhanging end.
[17] The therapeutic agent according to [15], wherein the guide strand and the passenger strand are nucleic acid molecules linked via a linker.
[18] The method, the nucleic acid, or the use according to [5] to [8], wherein the nucleic acid suppressing the expression of the NDR2 gene is a nucleic acid molecule comprising a guide strand and a passenger strand, the guide strand and the passenger strand are each 15- to 50-nt, and a sequence suppressing expression contained in the guide strand is a nucleic acid complementary to the NDR2 gene.
[19] The method, the nucleic acid, or the use according to [18], wherein the guide strand and/or the passenger strand is a nucleic acid molecule having an overhanging end.
[20] The method, the nucleic acid, or the use according to [18], wherein the guide strand and the passenger strand are nucleic acid molecules linked via a linker.
[21] The therapeutic agent, the method, the nucleic acid, or the use according to [3], [5] to [9], and [14], wherein the antisense nucleic acid is administered while encapsulated in a liposome, a glycolic acid copolymer (PLGA) microsphere, a lipid microsphere, a polymeric micelle, a gel agent, or an exosome drug delivery carrier.
[22] A therapeutic agent for fibrosis, comprising a low molecular weight compound having an NDR2 inhibitory activity.
[23] A method for treating fibrosis, comprising administering a low molecular weight compound having an NDR2 inhibitory activity to a subject.
[24] A low molecular weight compound having an NDR2 inhibitory activity for use in the treatment of fibrosis.
[25] Use of a low molecular weight compound having an NDR2 inhibitory activity in the manufacture of a therapeutic agent for fibrosis.
[26] The therapeutic agent, the method, the low molecular weight compound, or the use according to [22] to [25], wherein the fibrosis is pulmonary fibrosis, hepatic fibrosis, or kidney fibrosis.

### Advantageous Effects of Invention

According to the present invention, a novel therapeutic agent for fibrosis can be provided.

### Brief Description of Drawings

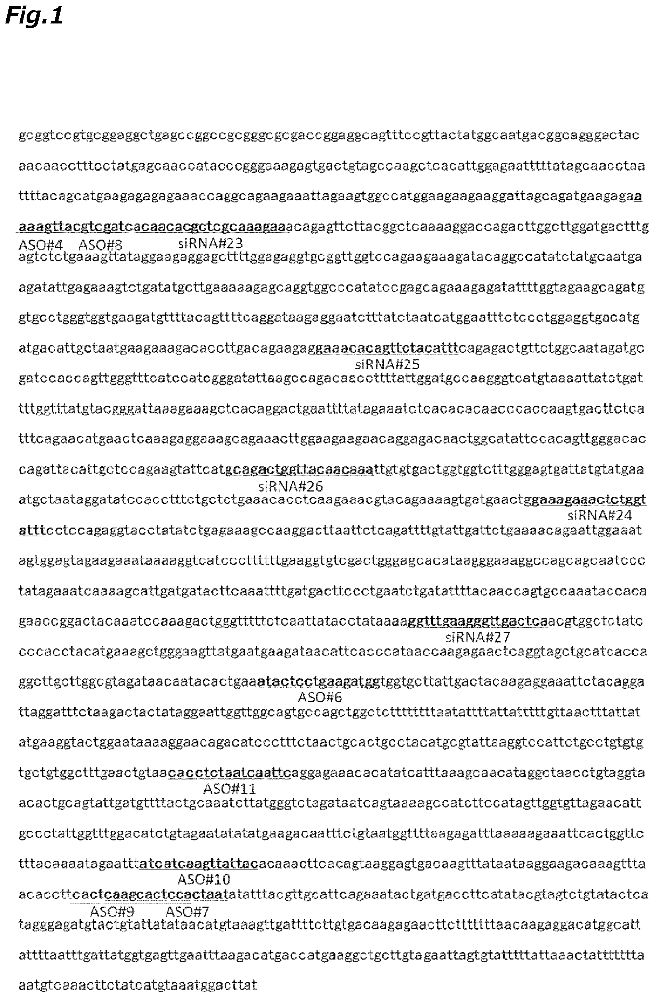

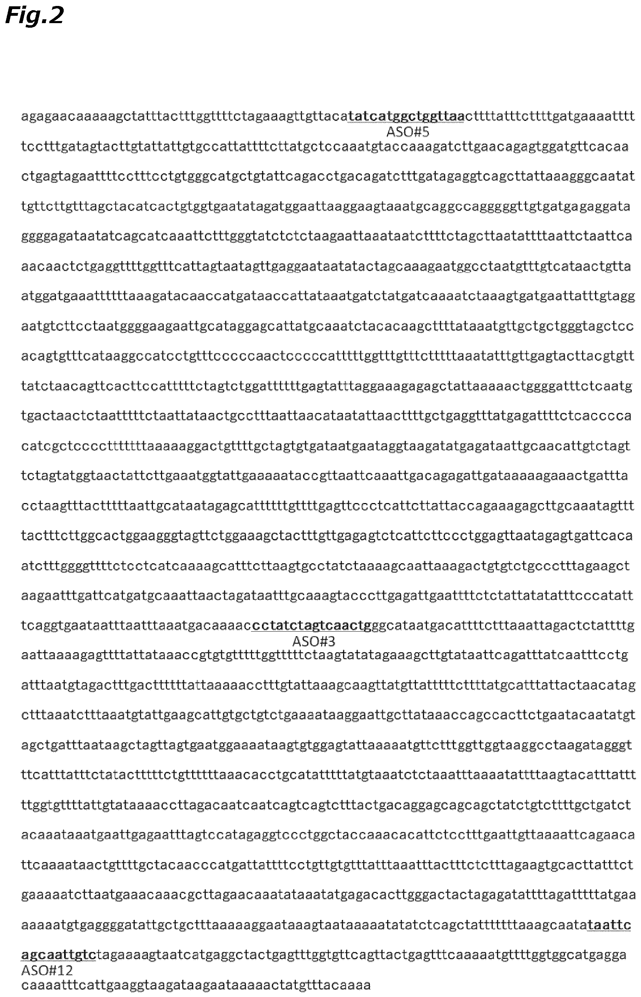

FIG. 1 is a view illustrating a sequence from the 5' end to the 2,400th nucleotide of mRNA of human-derived NDR2 gene and a target site of each of an antisense nucleic acid and siRNA described in the present disclosure. In the drawing, for example, ASO#4 indicates that the underlined sequence is a target sequence of the antisense nucleic acid shown in SEQ ID NO: 4. In addition, for example, siRNA#23 indicates that the underlined sequence is a target sequence of siRNA comprising a guide strand shown in SEQ ID NO: 23. Note that U (uracil) on the mRNA sequence is denoted as T (thymine). In addition, these notations are the same as those in FIGS. 2 to 4.
FIG. 2 is a view illustrating a nucleotide sequence starting from the 2,401th from the 5' end of mRNA of human-derived NDR2 gene and a target site of each of an antisense nucleic acid and siRNA described in the present disclosure.
FIG. 3 is a view illustrating a sequence from the 5' end to the 2,400th nucleotide of mRNA of mouse-derived NDR2 gene and a target site of each of an antisense nucleic acid and siRNA described in the present disclosure.
FIG. 4 is a view illustrating a nucleotide sequence starting from the 2,401th from the 5' end of mRNA of mouse-derived NDR2 gene and a target site of each of an antisense nucleic acid and siRNA described in the present disclosure.
FIG. 5 is a graph showing the transcript amount of NDR2 gene when each antisense nucleic acid was introduced into human IPF pulmonary fibroblasts.
FIG. 6 is a graph showing the amount of secreted collagen when NDR2 was knocked down by each antisense nucleic acid in human IPF pulmonary fibroblasts.
FIG. 7 is a graph showing the transcript amount of NDR2 gene when antisense nucleic acids having different concentrations were introduced into human IPF pulmonary fibroblasts.
FIG. 8 is a graph showing the amount of secreted collagen when NDR2 was knocked down by antisense nucleic acids having different concentrations in human IPF pulmonary fibroblasts.
FIG. 9 is a graph showing the transcript amount of NDR2 gene when each siRNA was introduced into human IPF pulmonary fibroblasts.
FIG. 10 is a graph showing the transcript amount of NDR2 gene when ON-TARGET plus pool siRNA was introduced as an siRNA into human IPF pulmonary fibroblasts.
FIG. 11 is a graph showing the transcript amount of NDR2 gene when Silencer Select siRNA was introduced as an siRNA into human IPF pulmonary fibroblasts.
FIG. 12 is a graph showing the amount of secreted collagen when NDR2 was knocked down by each siRNA in human IPF pulmonary fibroblasts.
FIG. 13 is a graph showing the amount of secreted collagen when NDR2 was knocked down by ON-TARGET plus pool siRNA in human IPF pulmonary fibroblasts.
FIG. 14 is a graph showing the amount of secreted collagen when NDR2 was knocked down by Silencer Select siRNA as an siRNA in human IPF pulmonary fibroblasts.
FIG. 15 is a graph showing the transcript amount of NDR2 gene when each antisense nucleic acid was introduced into human hepatic stellate cells.
FIG. 16 is a graph showing the amount of secreted collagen when NDR2 was knocked down by each antisense nucleic acid in human hepatic stellate cells.
FIG. 17 is a graph showing the transcript amount of NDR2 gene when each siRNA was introduced into human hepatic stellate cells.
FIG. 18 is a graph showing the transcript amount of NDR2 gene when Silencer Select siRNA was introduced as an siRNA into human hepatic stellate cells.
FIG. 19 is a graph showing the amount of secreted collagen when NDR2 was knocked down by each siRNA in human hepatic stellate cells.
FIG. 20 is a graph showing the amount of secreted collagen when NDR2 was knocked down by Silencer Select siRNA as an siRNA in human hepatic stellate cells.
FIG. 21 is a graph showing the transcript amount of NDR2 gene when ON-TARGET plus pool siRNA was introduced as an siRNA into human renal fibroblasts.
FIG. 22 is a graph showing the amount of secreted collagen when NDR2 was knocked down by ON-TARGET plus pool siRNA in human renal fibroblasts.
FIG. 23 is a graph showing the transcript amount of NDR2 gene when an antisense nucleic acid was introduced into mouse pulmonary fibroblasts.
FIG. 24 is a graph showing the amount of secreted procollagen when NDR2 was knocked down by an antisense nucleic acid in mouse pulmonary fibroblasts.
FIG. 25 is a graph showing the transcript amount of NDR2 gene when each siRNA was introduced into mouse hepatic stellate cells.
FIG. 26 is a graph showing the amount of secreted collagen when NDR2 was knocked down by each siRNA in mouse hepatic stellate cells.
FIG. 27 is a graph showing the transcript amount of NDR2 gene in the mouse lung when an antisense nucleic acid suppressing expression of NDR2 gene was oropharyngeally aspirated to normal mice.
FIG. 28 is a graph showing the transcript amount of NDR2 gene in a mouse liver when an antisense nucleic acid suppressing expression of NDR2 gene was subcutaneously administered or administered via tail vein to normal mice.
FIG. 29 is a graph showing the transcript amount of NDR2 gene in a mouse liver when an siRNA suppressing expression of NDR2 gene was administered via tail vein to normal mice.
FIG. 30 is a graph showing the transcript amount of NDR1 gene in a mouse liver when an siRNA suppressing expression of NDR2 gene was administered via tail vein to normal mice.
FIG. 31 is a graph showing the results of Western blotting of NDR2 protein and CYP2E1 protein in a mouse liver when an siRNA suppressing expression of NDR2 gene was administered via tail vein to normal mice.
FIG. 32 is a graph showing values of NDR2 protein corrected by Vinculin in a mouse liver when an siRNA suppressing expression of NDR2 gene was administered via tail vein to normal mice.
FIG. 33 is a graph showing values of CYP2E1 protein corrected by Vinculin in a mouse liver when an siRNA suppressing expression of NDR2 gene was administered via tail vein to normal mice.
FIG. 34 is a graph showing the transcript amount of NDR2 gene in a mouse liver when an siRNA suppressing expression of NDR2 gene was administered via tail vein to carbon tetrachloride (CCl₄)-induced hepatic fibrogenesis model mice.
FIG. 35 is a graph showing the transcript amount of CYR61 gene in a mouse liver when an siRNA suppressing expression of NDR2 gene was administered via tail vein to carbon tetrachloride (CCl₄)-induced hepatic fibrogenesis model mice.
FIG. 36 is a graph showing the transcript amount of CTGF gene in a mouse liver when an siRNA suppressing expression of NDR2 gene was administered via tail vein to carbon tetrachloride (CCl₄)-induced hepatic fibrogenesis model mice.
FIG. 37 is a graph showing the transcript amount of TNF gene in a mouse liver when an siRNA suppressing expression of NDR2 gene was administered via tail vein to carbon tetrachloride (CCl₄)-induced hepatic fibrogenesis model mice.
FIG. 38 is a graph showing the transcript amount of CCL2 gene in a mouse liver when an siRNA suppressing expression of NDR2 gene was administered via tail vein to carbon tetrachloride (CCl₄)-induced hepatic fibrogenesis model mice.
FIG. 39 is a graph showing the transcript amount of COL1A1 gene in a mouse liver when an siRNA suppressing expression of NDR2 gene was administered via tail vein to carbon tetrachloride (CCl₄)-induced hepatic fibrogenesis model mice.
FIG. 40 is a graph showing the transcript amount of TGF-β1 gene in a mouse liver when an siRNA suppressing expression of NDR2 gene was administered via tail vein to carbon tetrachloride (CCl₄)-induced hepatic fibrogenesis model mice.
FIG. 41 illustrates the results of Western blotting of αSMA protein in a mouse liver when an siRNA suppressing expression of NDR2 gene was administered via tail vein a to carbon tetrachloride (CCl₄)-induced hepatic fibrogenesis model mouse.
FIG. 42 is a graph showing the value of αSMA protein corrected by Vinculin in a mouse liver when an siRNA suppressing expression of NDR2 gene was administered via tail vein to carbon tetrachloride (CCl₄)-induced hepatic fibrogenesis model mice.

### Description of Embodiments

The terms herein used are used to mean as commonly used in the art unless otherwise stated. Additionally, in the present invention, "number of nucleotides" means, for example, "length", and can also be referred to as "nucleotide length". In the present invention, the range of the number of nucleotides discloses, for example, all of the positive integers falling within the range, and as a specific example, the description "1- to 4-nt" means the disclosure of all of "1-, 2-, 3-, 4-nt".

The present invention provides a therapeutic agent for fibrosis comprising a nucleic acid suppressing expression of NDR2 gene as an active ingredient. Hereinafter, the contents of the present invention will be described in detail.

### (1) Nucleic Acid Suppressing Expression of NDR2 Gene

The NDR2 protein is a serine/threonine kinase that is one of subtypes of Nuclear Dbf2-related kinases (NDR kinases) belonging to AGC family.

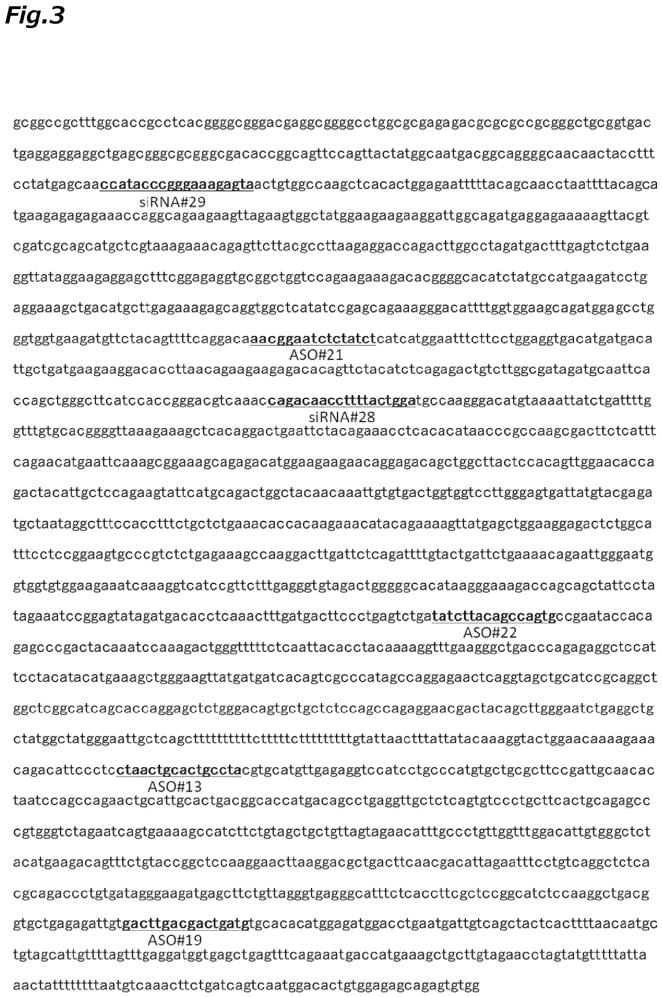

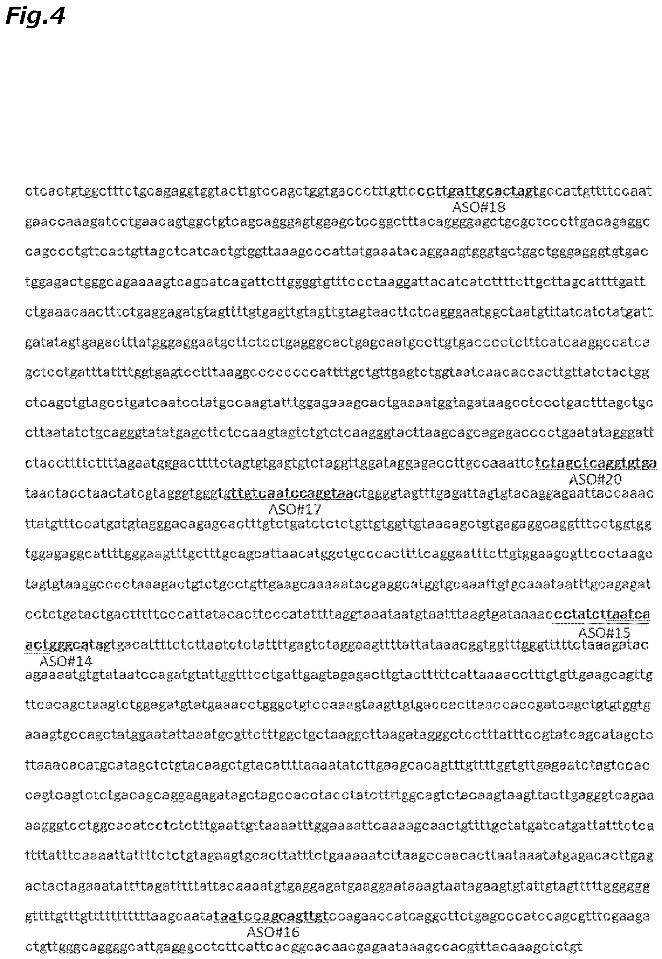

The NDR2 gene, which is a target of the present invention, is a gene derived from mammals, preferably a gene derived from humans or mice, and more preferably a gene derived from humans. For example, an mRNA sequence of one isoform of the NDR2 gene from humans (NCBI GenBank Accession No.: NM_015000.4) is shown in FIGS. 1 and 2 (SEQ ID NO: 1). In addition, for example, an mRNA sequence of one isoform of the NDR2 gene from mice (NCBI GenBank Accession No.: NM_172734.3) is shown in FIGS. 3 and 4 (SEQ ID NO: 2).

The mechanism of suppressing the expression of the NDR2 gene by the nucleic acid molecule is not particularly limited, and any mechanism may be used as long as the mechanism can down-regulate the expression. Suppression of the expression of the NDR2 gene can be confirmed by a decrease in production of a transcription product from the NDR2 gene, a decrease in production of a translation product from the NDR2 gene, a decrease in activity of a translation product, or the like.

Examples of the nucleic acid suppressing the expression of the NDR2 gene include an antisense nucleic acid of NDR2 mRNAs and siRNAs. The nucleic acid may be a single-strand nucleic acid or a double-strand nucleic acid.

Those skilled in the art can easily obtain an antisense nucleic acid and siRNA based on the nucleotide sequence of the NDR2 gene. A nucleic acid prepared based on the NDR2 mRNA sequence shown in SEQ ID NO: 1 (derived from humans) or SEQ ID NO: 2 (derived from mice) is preferable, and a nucleic acid prepared based on the NDR2 mRNA sequence shown in SEQ ID NO: 1 (derived from humans) is more preferable. For example, the nucleic acid can be prepared based on a translated region or a 3' untranslated region of the mRNA sequence of SEQ ID NO: 1. Specifically, the nucleic acid can be prepared based on a 59- to 1,450-nt DNA sequence or 1,451- to 4,957-nt DNA sequence from the 5' end of SEQ ID NO: 1. In addition, for example, the nucleic acid can be prepared based on a translated region or a 3' untranslated region of the mRNA sequence of SEQ ID NO: 2. Specifically, the nucleic acid can be prepared based on a 130- to 1,524-nt or 1,525- to 4,617-nt DNA sequence from the 5' end of SEQ ID NO: 2. In addition, for example, the nucleic acid can be prepared based on a precursor of mRNA of SEQ ID NO: 1 (NCBI GenBank Accession No.: NC_000012.12 REGION: 27244286..27325959) or a precursor of mRNA of SEQ ID NO: 2 (NC_000072.7 REGION: 146626428..146680312).

### (2) Antisense Nucleic Acid

The antisense nucleic acid is an oligonucleotide (antisense DNA and/or antisense RNA) that forms a double strand with a target mRNA in a sequence-specific manner and performs function inhibition such as splicing inhibition and translation inhibition, and exhibits an effect by introducing an antisense nucleic acid for the entire length or a part of the target mRNA into a cell.

Examples of a mechanism of inhibiting the expression of the antisense nucleic acid include:
1) steric inhibition of a translation initiation complex by directing to a region from the 5' cap site of mRNA to about 25-nt downstream of the initiation codon as a target sequence;
2) mRNA degradation via RNaseH with a single-strand DNA complementary to a target mRNA or pre-mRNA (mRNA precursor); and
3) splicing inhibition that directs to a boundary region between an exon and an intron of a pre-mRNA as a target sequence (mRNA maturation inhibition). The mechanism thereof is not particularly limited as long as it suppresses the expression of the NDR2 gene.

As the sequence contained in the antisense nucleic acid according to the present embodiment, it is preferable to select a sequence specific to the NDR2 gene. For example, a sequence that is complementary to the NDR2 gene and is not complementary to the NDR1 gene (RefSeq database: NM_001305102.2 and/or NM_007271.4), or that is complementary to the NDR2 gene and has one or more (for example, 1- to 3-nt) nucleotides in the region that are not complementary (mismatched) to the NDR1 gene can be selected. Increasing (for example, 4-nt or more, and preferably 5- to 7-nt) nucleotides that are complementary to the NDR2 gene and are not complementary (mismatched) to the NDR1 gene is useful in avoiding an off-target effect.

The number of nucleotides contained in the antisense nucleic acid according to the present embodiment is not particularly limited as long as the effect of suppressing the expression of the NDR2 gene is exhibited, and may be, for example, 10- to 40-nt, 10- to 35-nt, 12- to 30-nt, 13- to 30-nt, or 13- to 21-nt.

The antisense nucleic acid suppressing the gene expression of NDR2 can be obtained, for example, according to an antisense nucleic acid sequence design system such as Pfizer RNAi Enumeration and Design (PFRED) based on the cDNA sequence information of the mature mRNA of NDR2.

The antisense nucleic acid preferably contains a modified nucleotide residue in view of binding stability with RNA (such as Tm value), mismatch sequence recognition capability, nuclease resistance, RNaseH activity, and the like.

Specific examples of the antisense nucleic acid suppressing the expression of the NDR2 gene include the following single-strand nucleic acid molecules.
[a] Sequence having at least 90% sequence identity to a sequence of SEQ ID NO: 3.
[b] Sequence having at least 90% sequence identity to a sequence of SEQ ID NO: 4.
[c] Sequence having at least 90% sequence identity to a sequence of SEQ ID NO: 5.
[d] Sequence having at least 90% sequence identity to a sequence of SEQ ID NO: 6.
[e] Sequence having at least 90% sequence identity to a sequence of SEQ ID NO: 7.
[f] Sequence having at least 90% sequence identity to a sequence of SEQ ID NO: 8.
[g] Sequence having at least 90% sequence identity to a sequence of SEQ ID NO: 9.
[h] Sequence having at least 90% sequence identity to a sequence of SEQ ID NO: 10.
[i] Sequence having at least 90% sequence identity to a sequence of SEQ ID NO: 11.
[j] Sequence having at least 90% sequence identity to a sequence of SEQ ID NO: 12.
[k] Sequence having at least 90% sequence identity to a sequence of SEQ ID NO: 13.
[l] Sequence having at least 90% sequence identity to a sequence of SEQ ID NO: 14.
[m] Sequence having at least 90% sequence identity to a sequence of SEQ ID NO: 15.
[n] Sequence having at least 90% sequence identity to a sequence of SEQ ID NO: 16.
[o] Sequence having at least 90% sequence identity to a sequence of SEQ ID NO: 17.
[p] Sequence having at least 90% sequence identity to a sequence of SEQ ID NO: 18.
[q] Sequence having at least 90% sequence identity to a sequence of SEQ ID NO: 19.
[r] Sequence having at least 90% sequence identity to a sequence of SEQ ID NO: 20.
[s] Sequence having at least 90% sequence identity to a sequence of SEQ ID NO: 21.
[t] Sequence having at least 90% sequence identity to a sequence of SEQ ID NO: 22.

The sequence identity of the sequence contained in the antisense nucleic acid with the sequences of SEQ ID NOs: 3 to 22 may be 90% or more, 92% or more, 95% or more, or 98% or more, and may be 100%. Various modifications may be added to the antisense nucleic acids [a] to [t] as long as the action of suppressing the expression of the NDR2 gene is not lost. The various modifications may be present in the sequences of SEQ ID NOs: 3 to 22 or may be present outside of the sequences. Details of the modified nucleotides and the like will be described below.

The antisense nucleic acid suppressing the gene expression of NDR2 can be easily obtained by a known method based on the nucleotide sequence of the NDR2 gene. For example, the antisense nucleic acid can be appropriately designed based on mRNA sequence information of the NDR2 gene such as the sequence of the NDR2 mRNA shown in SEQ ID NO: 1 or SEQ ID NO: 2, and can be synthesized by a conventionally known method. Examples of the synthesis method include a synthesis method by a genetic engineering method and a chemical synthesis method. Examples of the genetic engineering method include an in vitro transcription synthesis method, a method using a vector, and a method using a PCR cassette. The vector is not particularly limited, and examples thereof include a non-viral vector such as a plasmid and a viral vector. The chemical synthesis method is not particularly limited, and examples thereof include a phosphoramidite method and an H-phosphonate method. As the chemical synthesis method, for example, a commercially available automatic nucleic acid synthesizer can be used. In addition, it is also possible to design and acquire a sequence of a gapmer-type antisense in which a crosslinked nucleic acid is introduced into both ends by providing the gene information of NDR2 to a manufacturer (for example, Qiagen N.V. and Hokkaido System Science CO., Ltd.).

### (3) siRNAs

A small interfering RNA (siRNA), which is one of the nucleic acids suppressing the expression of the NDR2 gene, will be described below.

An siRNA is a nucleic acid molecule comprising a guide strand (antisense strand) that pairs with a target gene and a passenger strand (sense strand) that forms a double strand with the guide strand. After siRNAs are incorporated into a complex called an RNA-inducing silencing complex (RISC) with AGO (Argonaute) protein as a core in the cell, the sense strand is degraded by AGO, and the guide strand remains in RISC. A seed region of the guide strand (a 7-nt region at positions 2 to 8 from the 5' end of the guide strand) is considered to have an important function in recognizing the target sequence, and a seed region specific to the target gene can be selected for the purpose of avoiding the off-target effect. For example, it is preferable to select a sequence specific to NDR2 mature mRNA (RefSeq database: NM_015000.4 and/or NM_172734.3) also for the seed region of the nucleic acid that is the active ingredient of the present invention. For example, a nucleic acid that is complementary to the NDR2 mature gene and is not complementary to the NDR1 mature gene (RefSeq database: NM_001305102.2 and/or NM_007271.4), or that is complementary to the NDR2 mature gene and has one or more (for example, 1- to 3-nt) nucleotides in the region that are not complementary (mismatched) to the NDR2 mature gene as a seed region can be selected. Also for the full-length sequence, for example, increasing the number of nucleotides that are complementary to the NDR2 mature mRNA and are not complementary (mismatched) to the NDR1 mature mRNA (for example, 4-nt or more, and preferably 5- to 7-nt) is useful in avoiding the off-target effect. The number of nucleotides in the sequence suppressing expression contained in the guide strand is, for example, 15- to 30-nt, preferably 19- to 25-nt, more preferably 19- to 23-nt, still more preferably 21-, 22-, and 23-nt, and particularly preferably 23-nt.

The sequence suppressing expression may further have an additional sequence on the 3' side to form an overhanging end. The number of nucleotides in the additional sequence is, for example, 1- to 11-nt and preferably 1- to 4-nt. The additional sequence may be a ribonucleotide residue or a deoxyribonucleotide residue.

The number of nucleotides in the guide strand is, for example, 15- to 50-nt, preferably 17- to 30-nt, more preferably 19- to 25-nt, still more preferably 19- to 23-nt, much more preferably 21-, 22-, and 23-nt, and particularly preferably 23-nt.

The number of nucleotides in the passenger strand is, for example, 15- to 50-nt, preferably 17- to 30-nt, more preferably 19- to 25-nt, still more preferably 19- to 23-nt, much more preferably 21-, 22-, and 23-nt, and particularly preferably 21-nt.

In the passenger strand, the region exhibiting complementarity with the guide strand is, for example, 15- to 50-nt, preferably 17- to 30-nt, more preferably 19-to 25-nt, and still more preferably 19- to 23-nt. The region may further have an additional sequence on the 3' side. The number of nucleotides in the additional sequence is, for example, 1- to 11-nt, and preferably 1- to 4-nt, and the additional sequence may be a ribonucleotide residue or a deoxyribonucleotide residue. The passenger strand may be, for example, complementary to a region exhibiting complementarity of the guide strand, or may be not complementary to one or several nucleotides, and is preferably complementary. The one nucleotide or the several nucleotides is, for example, 1- to 3-nt, and preferably 1-nt or 2-nt.

An siRNA suppressing the gene expression of NDR2 can be obtained based on the cDNA sequence information of the NDR2 mature mRNA, for example, according to siRNA sequence design systems such as siSNIPER (registered trademark), siDirect (registered trademark) for drug discovery and diagnosis research, and PFRED.

An NDR2 siRNA is preferably an siRNA that specifically acts on NDR2, and examples thereof include the following double-strand nucleic acids.
(a) Double-strand nucleic acid molecule comprising a guide strand (antisense strand) comprising a sequence having at least 90% sequence identity to a sequence of SEQ ID NO: 23 and a passenger strand (sense strand) complementary to the guide strand
(b) Double-strand nucleic acid molecule comprising a guide strand (antisense strand) comprising a sequence having at least 90% sequence identity to a sequence of SEQ ID NO: 24 and a passenger strand (sense strand) complementary to the guide strand
(c) Double-strand nucleic acid molecule formed from a guide strand (antisense strand) containing a sequence having at least 90% sequence identity to a sequence of SEQ ID NO: 25 and a passenger strand (sense strand) complementary to the guide strand
(d) Double-strand nucleic acid molecule formed from a guide strand (antisense strand) containing a sequence having at least 90% sequence identity to a sequence of SEQ ID NO: 26 and a passenger strand (sense strand) complementary to the guide strand
(e) Double-strand nucleic acid molecule comprising a guide strand (antisense strand) comprising a sequence having at least 90% sequence identity to a sequence of SEQ ID NO: 27 and a passenger strand (sense strand) complementary to the guide strand
(f) Double-strand nucleic acid molecule comprising a guide strand (antisense strand) comprising a sequence having at least 90% sequence identity to a sequence of SEQ ID NO: 28 and a passenger strand (sense strand) complementary to the guide strand
(g) Double-strand nucleic acid molecule comprising a guide strand (antisense strand) comprising a sequence having at least 90% sequence identity to a sequence of SEQ ID NO: 29 and a passenger strand (sense strand) complementary to the guide strand

The sequence identity of the sequence contained in the passenger strand (sense strand) contained in the double-strand nucleic acid molecule with the sequences of SEQ ID NOs: 1 and 2 may be 90% or more, 92% or more, 95% or more, or 98% or more, and may be 100%.

In addition, the siRNA suppressing the expression of the NDR2 gene may be a single-strand nucleic acid molecule in which a hairpin RNA structure is formed by linking the 3' end of the passenger strand (sense strand) and the 5' end of the guide strand (antisense strand) via a linker sequence consisting of nucleotide residues and/or a linker having a non-nucleotide structure, or linking the 3' end of the guide strand (antisense strand) and the 5' end of the passenger strand (sense strand) via a linker sequence consisting of nucleotide residues and/or a linker having a non-nucleotide structure.

The length of the linker sequence consisting of the nucleotide residues is not particularly limited, and is preferably a length with which the passenger strand and the guide strand can form a double strand. The number of nucleotides in the linker sequence is, for example, 1-nt or more, preferably 2-nt or more, more preferably 3-nt or more, and is, for example, 100-nt or less, preferably 80-nt or less, and more preferably 50-nt or less. Specific examples of the number of nucleotides in the linker sequence are 1- to 100-nt, 2- to 80-nt, or 3- to 50-nt.

Examples of the linker having a non-nucleotide structure include chemical linkers such as a hexaethylene glycol linker, a poly(oxyphosphinico-oxy-1,3-propanediol) linker, an allyl linker, and a polyethylene glycol linker, and an amino linker having a carbamate structure. The length of the non-nucleotide structure linker is not particularly limited, and is preferably a length with which the passenger strand and the guide strand can form a double strand.

### (4) Nucleotide Residues Used in Nucleic Acids

In the present embodiment, the nucleotide residue used for the nucleic acid as an active ingredient includes a sugar, a base, and a phosphoric acid as components. Examples of the nucleotide residue include a ribonucleotide residue and a deoxyribonucleotide residue. The ribonucleotide residue has, for example, a ribose residue as a sugar, and includes adenine (A), guanine (G), cytosine (C), and uracil (U) as a base, and the deoxyribonucleotide residue has, for example, a deoxyribose residue as a sugar, and includes adenine (A), guanine (G), cytosine (C), and thymine (T) as a base.

Examples of the nucleotide residue include an unmodified nucleotide residue and a modified nucleotide residue. In the unmodified nucleotide residue, the respective components are, for example, the same or substantially the same as those naturally occurring, and preferably the same or substantially the same as those naturally occurring in the human body.

The modified nucleotide residue is, for example, a nucleotide residue obtained by modifying the unmodified nucleotide residue. In the modified nucleotide, for example, any component of the unmodified nucleotide residue may be modified. In the present invention, the "modification" is, for example, substitution, addition, and/or deletion of the component, and substitution, addition, and/or deletion of an atom and/or a functional group in the component, and can be referred to as "alteration". Examples of the modified nucleotide residue include a naturally occurring nucleotide residue and an artificially modified nucleotide residue. Regarding the naturally-derived modified nucleotide residues, for example, Limbach et al. (Limbach et al., 1994, Summary: the modified nucleosides of RNA, Nucleic Acids Res. 22: 2,183 to 2,196) can be referred to. The modified nucleotide residue may be, for example, a residue of an alternative of the nucleotide.

Examples of the modification of the nucleotide residue include modification of a ribose-phosphate skeleton (hereinafter, referred to as a "ribophosphate skeleton"). In the ribophosphate skeleton, for example, a ribose residue can be modified. In the ribose residue, for example, the 2'-position carbon can be modified, and specifically, a hydroxyl group bound to, for example, the 2'-position carbon can be substituted with hydrogen, fluoro, or the like. By substituting the hydroxyl group of the 2'-position carbon with hydrogen, the ribose residue can be substituted with deoxyribose. In addition, modification can also be achieved by introducing a methyl group, a methoxyethyl group, or the like into the 2'-position hydroxyl group, and examples thereof include 2'-F, 2'-O-methyl (2'-OMe), and 2'-OMethoxyethyl (2'-MOE). The ribose residue can be substituted with, for example, a stereoisomer, and may be substituted with, for example, an arabinose residue.

The ribophosphate skeleton may be substituted with, for example, a non-ribophosphate skeleton having a non-ribose residue and/or a non-phosphate. Examples of the non-ribophosphate skeleton include uncharged forms of the ribophosphate skeleton. Examples of an alternative to the nucleotide substituted with the non-ribophosphate skeleton include morpholino, cyclobutyl, and pyrrolidine. Other examples of the alternative include an artificial nucleic acid monomer residue. Specific examples thereof include PNA (peptide nucleic acid), 2',4'-BNA [Bridged Nucleic Acid, also known as Locked Nucleic Acid (LNA)] which is a crosslinked artificial nucleic acid, AmNA, ENA (2'-O,4'-C-Ethylenebridged Nucleic Acids), GuNA, and scpBNA. When the nucleic acid according to the present embodiment is an antisense nucleic acid using the mechanism of RNA degradation by RNaseH, the nucleic acid is preferably an antisense nucleic acid into which an artificial nucleic acid is introduced, more preferably a nucleic acid into which an artificial nucleic acid is introduced into both ends of a sequence, and particularly preferably an antisense nucleic acid into which an artificial nucleic acid is introduced only into both ends of a sequence. This is because an antisense nucleic acid in which an artificial nucleic acid is introduced only into both ends of the sequence is used, such that RNA degradation by RNaseH occurs while maintaining the characteristics of the artificial nucleic acid, and a high antisense effect can be expected.

On the other hand, when the nucleic acid according to the present embodiment is an antisense nucleic acid using the mechanism of exon skipping induction, in order to prevent the target RNA from being cleaved, it is preferable that the nucleic acid is designed so that RNA degradation by RNaseH does not occur as in the case of a mixmer or an all-modified oligonucleotide.

In the ribophosphate skeleton, for example, a phosphate group can be modified. In the ribophosphate skeleton, the phosphate group closest to the sugar residue is called an α-phosphate group. The α-phosphate group is negatively charged and the charges are uniformly distributed over two oxygen atoms that are not linked to the sugar residue. Among the four oxygen atoms in the α-phosphate group, two oxygen atoms that are not linked to the sugar residue in a phosphodiester bond between the nucleotide residues are hereinafter also referred to as "non-linking oxygens". On the other hand, in the phosphodiester bond between the nucleotide residues, two oxygen atoms that are linked to the sugar residue are hereinafter referred to as "linking oxygens". The α-phosphate group is preferably subjected to, for example, uncharged modification or modification in which the charge distribution between the non-linking atoms is asymmetric.

In the phosphate group, for example, the non-linking oxygen may be substituted. The oxygen can be substituted with, for example, any atom of S (sulfur), Se (selenium), B (boron), C (carbon), H (hydrogen), N (nitrogen), and OR (R is, for example, an alkyl group or an aryl group), and substitution with S is preferable. For example, both of the non-linking oxygen atoms are preferably substituted, and more preferably, both of the non-linking oxygen atoms are substituted with S. Examples of the modified phosphate group include phosphorothioate, phosphorodithioate, phosphoroselenate, boranophosphate, boranophosphate ester, hydrogen phosphonate, phosphoramidate, alkyl or aryl phosphonate, and phosphotriester, and among them, phosphorodithioate in which both of the two non-linking oxygens are substituted with S is preferable.

In the phosphate group, for example, the linking oxygen may be substituted. The oxygen can be substituted with, for example, any atom of S (sulfur), C (carbon), and N (nitrogen), or borane (BH3). Examples of the modified phosphate group include a crosslinked phosphoramidate substituted with N, a crosslinked phosphorothioate substituted with S, and a crosslinked methylene phosphonate substituted with C. The phosphorothioate modification (S-formation) in which the oxygen atom is substituted with an S (sulfur) atom is a modification of a phosphate diester bond linking the nucleic acid and the nucleic acid, and the phosphate diester bond in the sequence is modified to a phosphorothioate bond. This is preferable because improvement of nuclease resistance can be expected. The substitution of the linking oxygen may be performed, for example, in at least one of the 5'-terminal nucleotide residue and the 3'-terminal nucleotide residue of the nucleic acid according to the present embodiment, or may be performed in both. In addition, for example, all phosphate diester bonds in the nucleic acid according to the present embodiment may be modified to phosphorothioate bonds.

The phosphate group may be substituted with, for example, the phosphorus-free linker. The linker includes, for example, siloxane, carbonate, carboxymethyl, carbamate, amide, thioether, ethylene oxide linker, sulfonate, sulfonamide, thioformacetal, formacetal, oxime, methylene imino, methylene methylimino, methylene hydrazo, methylene dimethyl hydrazo, and methylene oxymethylimino, and preferably includes a methylene carbonylamino group and a methylene methylimino group.

Examples of the modification of the terminal nucleotide residue include addition of other molecules. Examples of the other molecules include functional molecules such as a labeling substance and a protecting group as described above. Examples of the protecting group include S (sulfur), Si (silicon), B (boron), and an ester-containing group.

In the nucleotide residue, the base is not particularly limited. The base may be, for example, a natural base or a non-natural base. The base may be, for example, natural or synthetic. As the base, for example, a general base, a modified analog thereof, or the like can be used.

### (5) Therapeutic Agent for Fibrosis

The therapeutic agent for fibrosis according to the present embodiment is a therapeutic agent for hepatic fibrosis, hepatic cirrhosis, viral hepatitis, autoimmune hepatitis, primary biliary cholangitis, non-alcoholic steatohepatitis, alcoholic liver disease, primary sclerosing cholangitis, hemochromatosis, Wilson's disease, α1-antitrypsin deficiency, non-viral congestive hepatic cirrhosis, drug-induced hepatic disorder, pancreatitis, pancreatic fibrosis, retinal fibrosis, vocal cord scarring, vocal cord mucosal fibrosis, laryngeal fibrosis, pulmonary fibrosis, interstitial pneumonia, idiopathic pulmonary fibrosis, non-specific interstitial pneumonia, idiopathic organizing pneumonia, desquamative interstitial pneumonia, respiratory bronchiolitis-associated interstitial pneumonia, acute interstitial pneumonitia, lymphocytic interstitial pneumonia, sarcoidosis, chronic eosinophilic pneumonitis, acute eosinophilic pneumonia, lymphangioleiomyomatosis, pulmonary alveolar proteinosis, Hermansky-Pudlak syndrome, pulmonary Langerhans cell histiocytosis, siderosis, amyloidosis, alveolar microlithiasis, hypersensitivity pneumonitis, pneumoconiosis, infectious lung disease, drug-induced pneumonitis, radiation pneumonia, cystic fibrosis, myelofibrosis, cardiac fibrosis, heart failure, kidney fibrosis, chronic renal failure, diabetic nephropathy, chronic glomerulonephritis, malignant nephrosclerosis, polycystic kidney disease, drug-induced kidney disease, retroperitoneal fibrosis, collagen disease, scleroderma, dyskeratosis congenita, renal systemic fibrosis, and a wide range of diseases associated with fibrosis including airway fibrogenesis, intestinal fibrogenesis, urinary bladder fibrogenesis, prostatic fibrogenesis, and dermal fibrogenesis. The therapeutic agent for fibrosis is preferably a therapeutic agent for hepatic fibrosis, hepatic cirrhosis, pulmonary fibrosis, interstitial pneumonia, kidney fibrosis, or chronic renal failure, and more preferably pulmonary fibrosis, hepatic fibrosis, or kidney fibrosis.

An administration method of the therapeutic agent for fibrosis according to the present embodiment is not particularly limited, and is preferably parenteral administration such as inhalation, intravenous administration (intravenous injection), intramuscular administration, subcutaneous administration, intradermal administration, intrathecal administration, and transdermal administration. The therapeutic agent for fibrosis according to the present embodiment is preferably administered to a subject in need of the therapeutic agent for fibrosis, for example, a subject who develops diseases associated with fibrosis described above and a subject determined to have a high risk of onset of the diseases. The subject may be a human or a non-human animal. A dosage of the nucleic acid molecule according to the present embodiment in the method according to the present embodiment is not particularly limited as long as it is a therapeutically effective amount for the disease, and varies depending on the type of the disease, the degree of severity, age, body weight, administration route, and the like, but may be usually about 0.0001 to about 100 mg/kg by body weight, for example, about 0.001 to about 10 mg/kg by body weight, and preferably about 0.005 to about 5 mg/kg by body weight per once for an adult. The amount can be administered, for example, at intervals of three times a day to once a month, and preferably once a day to a week.

The therapeutic agent for fibrosis according to the present embodiment is usually formulated as an appropriate pharmaceutical composition together with a pharmaceutically acceptable carrier and administered in an oral or parenteral form.

The antisense nucleic acid or siRNA according to the present embodiment is preferably administered while encapsulated in a liposome, a glycolic acid copolymer (PLGA) microsphere, a lipid microsphere, a polymeric micelle, a gel agent, or an exosome drug delivery carrier.

### (6) Low Molecular Weight Compound Having NDR2 Inhibitory Activity

In addition to the nucleic acid suppressing the expression of the NDR2 gene, a low molecular compound having an NDR2 inhibitory activity may also be an active ingredient of the therapeutic agent for fibrosis. A low molecular weight compound means a compound having a molecular weight of less than 1,000.

Hereinafter, the present invention will be described in detail with reference to Examples and the like, but the present invention is not limited thereto. Note that, in the present Examples, "antisense oligonucleotide" and "ASO" have the same meaning as "antisense nucleic acid". In addition, in Examples, for example, #3 represents an antisense nucleic acid shown in SEQ ID NO: 3 or an siRNA comprising a guide strand shown in SEQ ID NO: 3. In addition, unless otherwise specified, the nucleic acid control uses ON-TARGET plus Control Pool Non-Targeting siRNA (D-001810-10-05, Dharmacon Inc.), Antisense LNA GapmeR Negative control A (339515LG00000002-DDA, Qiagen N.V), Ambion in vivo Negative Control siRNA (4459405, Thermo Fisher Scientific, Inc.), or Silenser select Nontarget siRNA (4390846, Thermo Fisher Scientific, Inc.). In addition, the cells are cultured under conditions of 37°C and 5% CO₂ unless otherwise noted.

### [Examples]

### [Example 1: NDR2 Knockdown Using ASO in Human IPF Pulmonary Fibroblasts]

The influence of ASO on the expression of NDR2 in human pulmonary fibroblast cell line LL29 cells (ATCC) established from the lung of an IPF patient was analyzed.

To LL29 cells, ASO (Antisense LNA GapmeR, Qiagen N.V.) for human NDR2 was transfected using Lipofectamine RNAiMAX (Thermo Fisher Scientific, Inc.) according to Lipofectamine RNAiMAX Reagent Protocol. For preparation of an ASO transfection solution, an RNAiMAX Transfection Reagent was mixed with 50 µL of an Opti-MEM solution so as to have a ratio of 2 µL, thereby preparing an RNAiMAX preparation solution. A preparation solution (ASO diluent) obtained by diluting ASO with Opti-MEM was mixed so as to be equal in volume to the RNAiMAX preparation solution, and 100 µL of the mixture was added to each well of a 24-well plate. Then, 500 µL of the cell suspension was seeded so that the LL29 cells were 2.5 × 10⁴ cells/well, and the cells were allowed to stand in a CO₂ incubator (final concentration of ASO: 30 nM). 48 hours after transfection, the medium was changed to F-12K medium containing 0.2% FBS. 24 hours after medium change, human TGF-β1 protein (PeproTech, Inc.) was added so as to have a final concentration of 3 ng/mL. RNAs were extracted from the cells 24 hours after TGF-β1 stimulation using RNeasy 96 Kit (Qiagen N.V.). The RNAs thus obtained were subjected to a reverse transcription reaction using High Capacity cDNA Reverse Transcription Kit (Applied Biosystems(R)) to obtain cDNAs. Using the cDNAs thus obtained as a template, real-time PCR was performed using Taqman Gene Expression Assays (Thermo Fisher Scientific, Inc.), TaqMan Fast Universal PCR master mix (Thermo Fisher Scientific, Inc.), and Applied Biosystems 7500 Fast Real-Time PCR Systems (Applied Biosystems(R)), and the influence of NDR2 knockdown on the transcript amount of NDR2 gene was examined. The transcript amount of the NDR2 gene was calculated by dividing the measured value in NDR2 Taqman Gene Expression Assays (Hs00960027_m1, Thermo Fisher Scientific, Inc.) by the measured value in GAPDH Taqman Gene Expression Assays (Hs02786624_g1).

For the sequence of ASO, Antisense LNA GapmeR (SEQ ID NOs: 3 to 5) was used.

### <Antisense LNA GapmeR>

ASONDR2:5'-CAGTTGACTAGATAGG-3' (SEQ ID NO: 3)
ASONDR2:5'-GATCGACGTAACTTTT-3' (SEQ ID NO: 4)
ASONDR2:5'-TTAACCAGCCATGATA-3' (SEQ ID NO: 5)

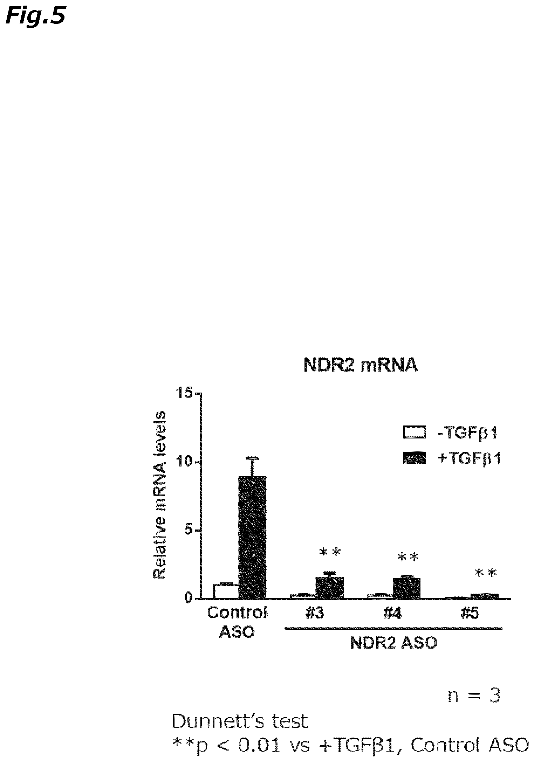

FIG. 5 illustrates the results of real-time PCR of NDR2 when Antisense LNA GapmeR for NDR2 was introduced into LL29 cells. The transcript amount of the NDR2 gene was suppressed by the introduction of NDR2 ASO. Therefore, it was shown that the expression of the target gene was efficiently suppressed by the NDR2 ASO used.

### [Example 2: Influence of NDR2 Knockdown on Collagen Secretion in Human IPF Pulmonary Fibroblasts]

In order to examine the possibility that NDR2 regulates collagen secretion in human pulmonary fibroblast cell line LL29 cells (ATCC) established from the lung of an IPF patient, the amount of collagen secreted in cells transfected with NDR2 ASO was analyzed.

To LL29 cells, ASO (Antisense LNA GapmeR, Qiagen N.V.) for human NDR2 was transfected using Lipofectamine RNAiMAX (Thermo Fisher Scientific, Inc.) according to Lipofectamine RNAiMAX Reagent Protocol. For preparation of an ASO transfection solution, an RNAiMAX Transfection Reagent was mixed with 50 µL of an Opti-MEM solution so as to have a ratio of 2 µL, thereby preparing an RNAiMAX preparation solution. A preparation solution (ASO diluent) obtained by diluting ASO with Opti-MEM was mixed so as to be equal in volume to the RNAiMAX preparation solution, and 100 µL of the mixture was added to each well of a 24-well plate. Then, 500 µL of the cell suspension was seeded so that the LL29 cells were 2.5 × 10⁴ cells/well, and the cells were allowed to stand in a CO₂ incubator (final concentration of ASO: 30 nM). 48 hours after transfection, the medium was changed to F-12K medium containing 0.2% FBS. 24 hours after medium change, human TGF-β1 protein (PeproTech, Inc.) was added so as to have a final concentration of 3 ng/mL. 48 hours after TGF-β1 stimulation, 400 µL of the culture supernatant was added to and mixed with 1 mL of ice-cooled Sircol Dye Reagent (Biocolor Ltd) in a Protein LoBind tube (Eppendorf). The tube was attached to a small rotary incubator, mixing was performed at 25°C for 30 minutes, centrifugation (4°C, 12,000 x g, for 10 minutes) was performed, and then, the supernatant was removed. Then, 750 µL of a cooled Acid-Salt Wash Reagent (Biocolor Ltd) was added thereto, and after centrifugation (4°C, 12,000 x g, for 10 minutes), the supernatant was removed. Then, 250 µL of Sircol Collagen Assay ALKALI Reagent (Biocolor Ltd) was added, and mixing was performed. 200 µL of the mixed solution was transferred to a 96-well plate, and an absorbance at 555 nm was measured using an absorption microplate reader.

For the sequence of ASO, Antisense LNA GapmeR (SEQ ID NOs: 3 to 5) was used.

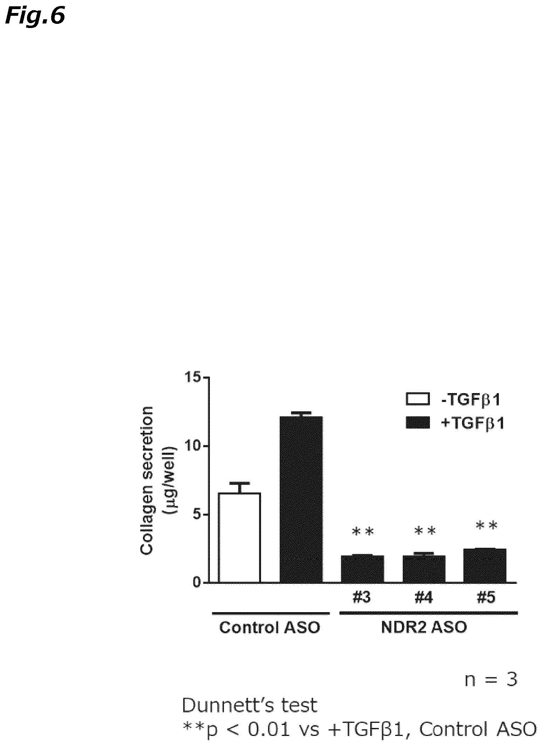

FIG. 6 illustrates the measurement results of collagen secretion when NDR2 was knocked down. By using ASO of NDR2, the gene expression level of NDR2 was reduced, and the collagen secretion amount increased by TGF-β1 was reduced. Therefore, it was shown that the collagen secretion induced by TGF-β1 in the human pulmonary fibroblast cell line was suppressed by NDR2 knockdown.

### [Example 3: ASO Concentration-dependent NDR2 Knockdown in Human IPF Pulmonary Fibroblasts]

To human pulmonary fibroblast cell line LL29 cells (ATCC) established from the lung of an IPF patient, ASO (Antisense LNA GapmeR, Qiagen N.V.) for human NDR2 prepared to have a final concentration of 10, 30 or 50 nM was transfected using Lipofectamine RNAiMAX (Thermo Fisher Scientific, Inc.) according to Lipofectamine RNAiMAX Reagent Protocol. For preparation of an ASO transfection solution, an RNAiMAX Transfection Reagent was mixed with 50 µL of an Opti-MEM solution so as to have a ratio of 2 µL, thereby preparing an RNAiMAX preparation solution. A preparation solution (ASO diluent) obtained by diluting ASO with Opti-MEM was mixed so as to be equal in volume to the RNAiMAX preparation solution, and 100 µL of the mixture was added to each well of a 24-well plate. Then, 500 µL of the cell suspension was seeded so that the LL29 cells were 2.5 × 10⁴ cells/well, and the cells were allowed to stand in a CO₂ incubator (prepared so that the final concentration of ASO was a target concentration). 48 hours after transfection, the medium was changed to F-12K medium containing 0.2% FBS. 24 hours after medium change, human TGF-β1 protein (PeproTech, Inc.) was added so as to have a final concentration of 3 ng/mL. RNAs were extracted from the cells 18 hours after TGF-β1 stimulation using RNeasy 96 Kit (Qiagen N.V.). The RNAs thus obtained were subjected to a reverse transcription reaction using High Capacity cDNA Reverse Transcription Kit (Applied Biosystems(R)). The transcript amount of NDR2 was measured using NDR2 Taqman Gene Expression Assays (Hs00960027_m1, Thermo Fisher Scientific, Inc.), and the relative ratio of the transcript amount of GAPDH as an internal standard to the value measured by GAPDH Taqman Gene Expression Assays (Hs02786624_g1) was taken as the transcript amount of gene.

For the sequence of ASO, Antisense LNA GapmeR (SEQ ID NO: 5) was used.

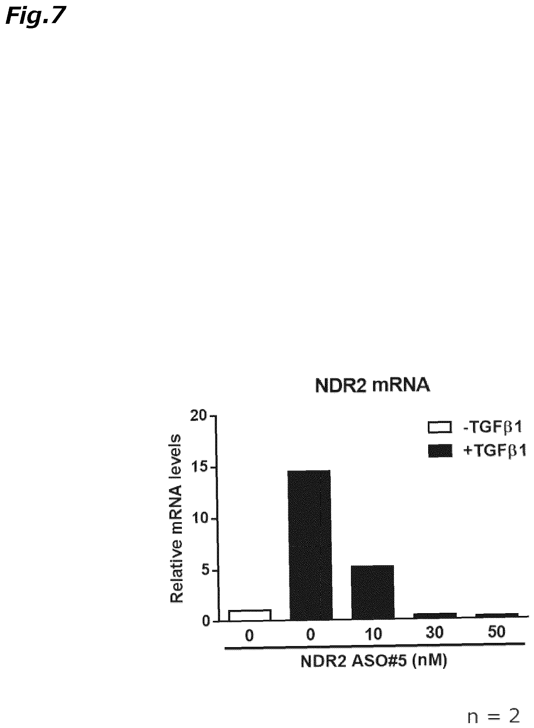

FIG. 7 illustrates the results of real-time PCR of NDR2 when Antisense LNA GapmeR for NDR2 was introduced into LL29 cells. The transcript amount of NDR2 gene was suppressed by the introduction of NDR2 ASO in a concentration-dependent manner. Therefore, it was shown that the expression of the target gene was suppressed by the NDR2 ASO used in a concentration-dependent manner.

### [Example 4: Influence of ASO Concentration-dependent NDR2 Knockdown on Collagen Secretion in Human IPF Pulmonary Fibroblasts]

To human pulmonary fibroblast cell line LL29 cells (ATCC) established from the lung of an IPF patient, ASO (Antisense LNA GapmeR, Qiagen N.V.) for human NDR2 prepared to have a final concentration of 10, 30 or 100 nM was transfected using Lipofectamine RNAiMAX (Thermo Fisher Scientific, Inc.) according to Lipofectamine RNAiMAX Reagent Protocol. For preparation of an ASO transfection solution, an RNAiMAX Transfection Reagent was mixed with 50 µL of an Opti-MEM solution so as to have a ratio of 2 µL, thereby preparing an RNAiMAX preparation solution. A preparation solution (ASO diluent) obtained by diluting ASO with Opti-MEM was mixed so as to be equal in volume to the RNAiMAX preparation solution, and 100 µL of the mixture was added to each well of a 24-well plate. Then, 500 µL of the cell suspension was seeded so that the LL29 cells were 2.5 × 10⁴ cells/well, and the cells were allowed to stand in a CO₂ incubator (prepared so that the final concentration of ASO was a target concentration). 48 hours after transfection, the medium was changed to F-12K medium containing 0.2% FBS. 24 hours after medium change, human TGF-β1 protein (PeproTech, Inc.) was added so as to have a final concentration of 3 ng/mL. 48 hours after TGF-β1 stimulation, 400 µL of the culture supernatant was added to and mixed with 1 mL of ice-cooled Sircol Dye Reagent (Biocolor Ltd) in a Protein LoBind tube (Eppendorf). The tube was attached to a small rotary incubator, mixing was performed at 25°C for 30 minutes, centrifugation (4°C, 12,000 x g, for 10 minutes) was performed, and then, the supernatant was removed. Then, 750 µL of a cooled Acid-Salt Wash Reagent (Biocolor Ltd) was added thereto, and after centrifugation (4°C, 12,000 x g, for 10 minutes), the supernatant was removed. Then, 250 µL of Sircol Collagen Assay ALKALI Reagent (Biocolor Ltd) was added, and mixing was performed. 200 µL of the mixed solution was transferred to a 96-well plate, and an absorbance at 555 nm was measured using an absorption microplate reader.

For the sequence of ASO, Antisense LNA GapmeR (SEQ ID NO: 5) was used.

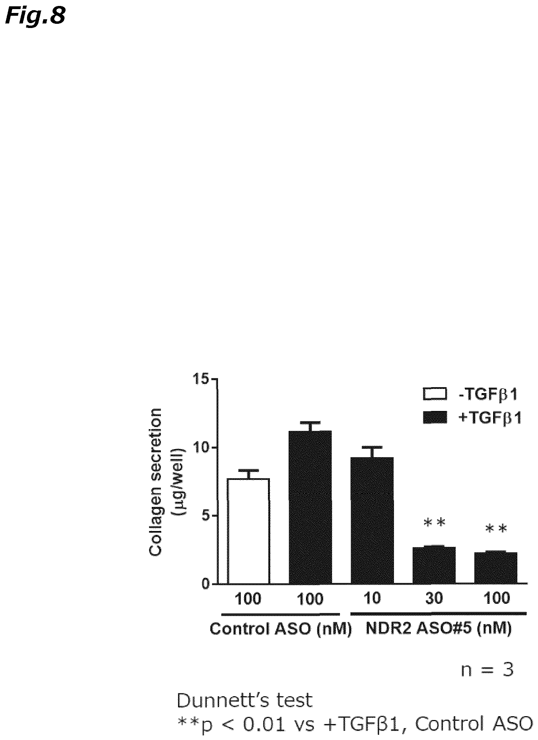

FIG. 8 illustrates the results of collagen secretion when Antisense LNA GapmeR for NDR2 was introduced into LL29 cells. The collagen secretion was suppressed in a concentration-dependent manner of NDR2 ASO. Therefore, it was shown that the collagen secretion was suppressed by concentration-dependent NDR2 knockdown ASO.

### [Example 5: NDR2 Knockdown Using siRNAs in Human IPF Pulmonary Fibroblasts]

The influence of siRNAs on the expression of NDR2 in human pulmonary fibroblast cell line LL29 cells (ATCC) established from the lung of an IPF patient was analyzed.

To LL29 cells, siRNAs for human NDR2 (ON-TARGET plus siRNA or ON-TARGET plus pool siRNA, Dharmacon Inc. or Silencer Select siRNA, Thermo Fisher Scientific, Inc.) were transfected using Lipofectamine RNAiMAX (Thermo Fisher Scientific, Inc.) according to Lipofectamine RNAiMAX Reagent Protocol. For preparation of an siRNA transfection solution, an RNAiMAX Transfection Reagent was mixed with 50 µL of an Opti-MEM solution so as to have a ratio of 2 µL, thereby preparing an RNAiMAX preparation solution. A preparation solution (siRNA diluent) obtained by diluting siRNAs with Opti-MEM was mixed so as to be equal in volume to the RNAiMAX preparation solution, and 100 µL of the mixture was added to each well of a 24-well plate. Then, 500 µL of a cell suspension was seeded so that the LL29 cells were 2.5 × 10⁴ cells/well, and the cells were allowed to stand in a CO₂ incubator (the final concentration of the ON-TARGET plus siRNA and the ON-TARGET plus pool siRNA was 30 nM, and the final concentration of the Silencer Select siRNA was 10 nM). 48 hours after transfection, the medium was changed to F-12K medium containing 0.2% FBS. 24 hours after medium change, human TGF-β1 protein (PeproTech, Inc.) was added so as to have a final concentration of 3 ng/mL. RNAs were extracted from the cells 48 hours or 24 hours after TGF-β1 stimulation using RNeasy 96 Kit (Qiagen N.V.). The RNAs thus obtained were subjected to a reverse transcription reaction using High Capacity cDNA Reverse Transcription Kit (Applied Biosystems(R)). The transcript amount of NDR2 was measured using NDR2 Taqman Gene Expression Assays (Hs00960027_m1, Thermo Fisher Scientific, Inc.), and the relative ratio of the transcript amount of GAPDH as an internal standard to the value measured by GAPDH Taqman Gene Expression Assays (Hs02786624_g1) was taken as the transcript amount of gene.

As the sequence of siRNA, ON-TARGET plus siRNA (SEQ ID NOs: 23 and 30), ON-TARGET plus siRNA (SEQ ID NOs: 24 and 31), ON-TARGET plus pool siRNA (the mixture of SEQ ID NOs: 23 to 26 and 30 to 33 in equal amounts), and Silencer Select siRNA (SEQ ID NOs: 27 and 34) were used. Note that, regarding the ON-TARGET plus siRNA, the presence or absence of an additional sequence is unknown.

### <ON-TARGET plus siRNA>

siNDR2:Sense:5'-ACAACACGCUCGCAAAGAA-3' (SEQ ID NO: 30)
Antisense:5'-UUCUUUGCGAGCGUGUUGU-3' (SEQ ID NO: 23)
siNDR2:Sense:5'-GAAAGAAACUCUGGUAUUU-3' (SEQ ID NO: 31)
Antisense:5'-AAAUACCAGAGUUUCUUUC-3' (SEQ ID NO: 24)
siNDR2:Sense:5'-GAAACACAGUUCUACAUUU-3' (SEQ ID NO: 32)
Antisense: 5'-AAAUGUAGAACUGUGUUUC-3' (SEQ ID NO: 25)
siNDR2:Sense:5'-GCAGACUGGUUACAACAAA-3' (SEQ ID NO: 33)
Antisense:5'-UUUGUUGUAACCAGUCUGC-3' (SEQ ID NO: 26)

### <Silencer Select siRNA>

siNDR2:Sense:5'-GGUUUGAAGGGUUGACUCAtt-3' (SEQ ID NO: 34)
Antisense:5'-UGAGUCAACCCUUCAAACCtt-3' (SEQ ID NO: 27)

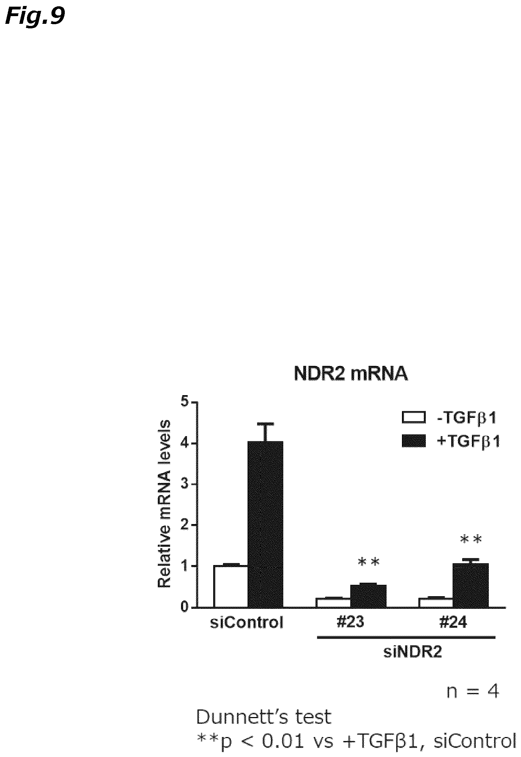

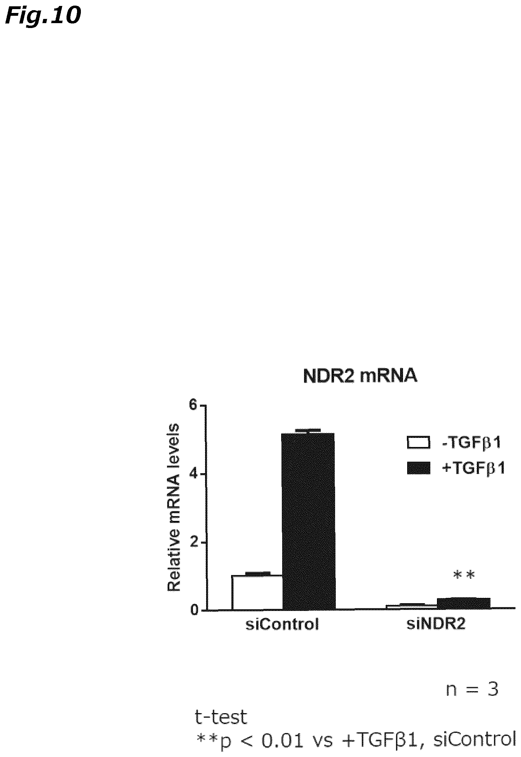

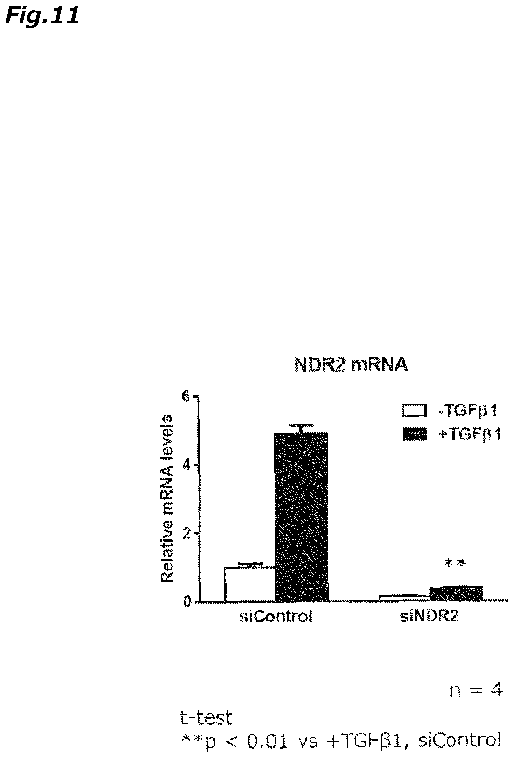

FIG. 9 illustrates the results of real-time PCR of NDR2 measured from RNAs extracted 48 hours after addition of TGF-β1 protein when ON-TARGET plus siRNA for NDR2 was introduced into LL29 cells. FIG. 10 illustrates the results of real-time PCR of NDR2 measured from RNAs extracted 24 hours after addition of TGF-β1 protein when ON-TARGET plus pool siRNA was introduced. FIG. 11 illustrates the results of real-time PCR of NDR2 measured from RNAs extracted 24 hours after addition of TGF-β1 protein when Silencer Select siRNA was introduced. The transcript amount of NDR2 gene induced by TGF-β1 was suppressed by the introduction of NDR2 siRNAs. Therefore, it was shown that the expression of the target gene was efficiently suppressed by the NDR2 siRNAs used.

### [Example 6: Influence of NDR2 siRNAs on Collagen Secretion in Human IPF Pulmonary Fibroblasts]

In order to examine the possibility that NDR2 regulates collagen secretion in human pulmonary fibroblast cell line LL29 cells (ATCC) established from the lung of an IPF patient, the amount of collagen secreted in cells transfected with NDR2 siRNAs was analyzed.

To LL29 cells, siRNAs for human NDR2 (ON-TARGET plus siRNA or ON-TARGET plus pool siRNA, (Dharmacon Inc.) or Silencer Select siRNA (Thermo Fisher Scientific, Inc.)) were transfected using Lipofectamine RNAiMAX (Thermo Fisher Scientific, Inc.) according to Lipofectamine RNAiMAX Reagent Protocol. For preparation of an siRNA transfection solution, an RNAiMAX Transfection Reagent was mixed with 50 µL of an Opti-MEM solution so as to have a ratio of 2 µL, thereby preparing an RNAiMAX preparation solution. A preparation solution (siRNA diluent) obtained by diluting siRNAs with Opti-MEM was mixed so as to be equal in volume to the RNAiMAX preparation solution, and 100 µL of the mixture was added to each well of a 24-well plate. Then, 500 µL of a cell suspension was seeded so that the LL29 cells were 2.5 × 10⁴ cells/well, and the cells were allowed to stand in a CO₂ incubator (the final concentration of the ON-TARGET plus siRNA and the ON-TARGET plus pool siRNA was 30 nM, and the final concentration of the Silencer Select siRNA was 10 nM). 48 hours after transfection, the medium was changed to F-12K medium containing 0.2% FBS. 24 hours after medium change, human TGF-β1 protein (PeproTech, Inc.) was added so as to have a final concentration of 3 ng/mL. 48 hours or 72 hours after TGF-β1 stimulation, 400 µL of the culture supernatant was added to and mixed with 1 mL of ice-cooled Sircol Dye Reagent (Biocolor Ltd) in a Protein LoBind tube (Eppendorf). The tube was attached to a small rotary incubator, mixing was performed at 25°C for 30 minutes, centrifugation (4°C, 12,000 x g, for 10 minutes) was performed, and then, the supernatant was removed. Then, 750 µL of a cooled Acid-Salt Wash Reagent (Biocolor Ltd) was added thereto, and after centrifugation (4°C, 12,000 x g, for 10 minutes), the supernatant was removed. Then, 250 µL of Sircol Collagen Assay ALKALI Reagent (Biocolor Ltd) was added, and mixing was performed. 200 µL of the mixed solution was transferred to a 96-well plate, and an absorbance at 555 nm was measured using an absorption microplate reader.

As the sequence of siRNA, ON-TARGET plus siRNA (SEQ ID NOs: 23 and 30), ON-TARGET plus siRNA (SEQ ID NOs: 24 and 31) and ON-TARGET plus pool siRNA (the mixture of SEQ ID NOs: 23 to 26 and 30 to 33 in equal amounts), or Silencer Select siRNA (SEQ ID NOs: 27 and 34) was used.

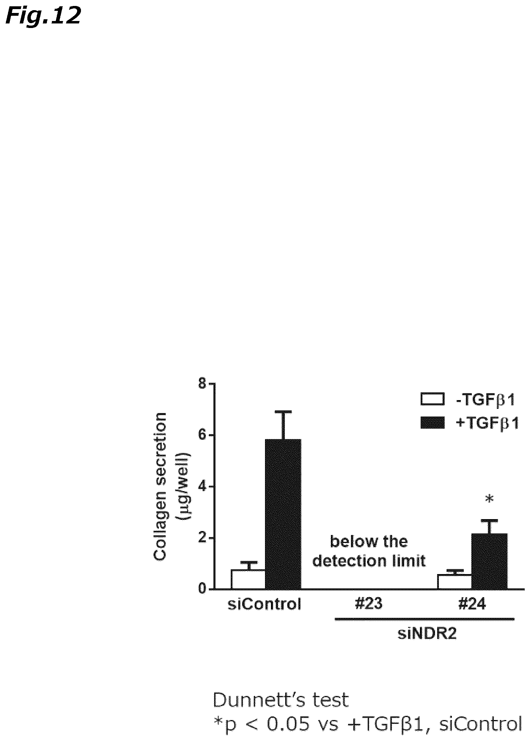

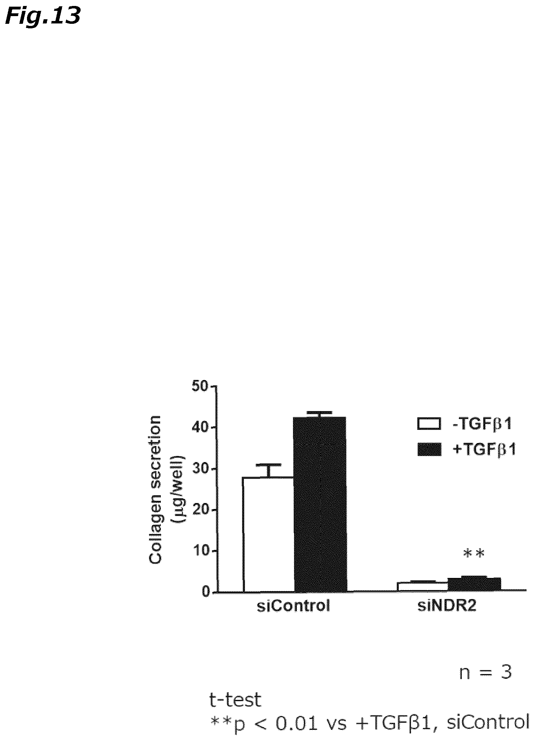

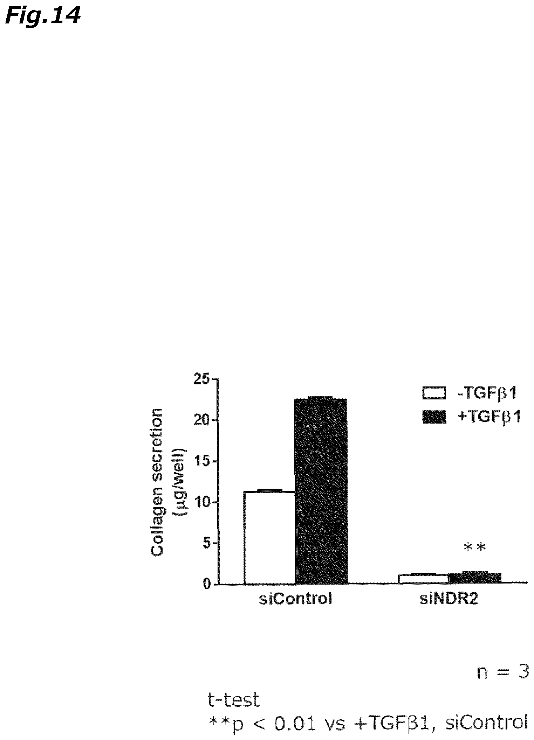

FIG. 12 illustrates the results of collagen secretion into the supernatant collected 48 hours after addition of TGF-β1 protein when ON-TARGET plus siRNA for NDR2 was introduced into LL29 cells. FIG. 13 illustrates the results of collagen secretion into the supernatant collected 72 hours after addition of TGF-β1 protein when ON-TARGET plus pool siRNA was introduced. FIG. 14 illustrates the results of collagen secretion into the supernatant collected 48 hours after addition of TGF-β1 protein when Silencer Select siRNA was introduced. The collagen secretion was suppressed by the introduction of NDR2 siRNAs. Therefore, it was shown that the collagen secretion was suppressed by NDR2 knockdown by NDR2 siRNAs.

### [Example 7: Knockdown by NDR2 ASO in Human Hepatic Stellate Cells]

The influence of ASO on the expression of NDR2 in human primary hepatic stellate cells (Samsara Sciences, Inc.) isolated from human liver was analyzed.

To human primary hepatic stellate cells, ASO (Antisense LNA GapmeR, Qiagen N.V.) for human NDR2 was transfected using Lipofectamine RNAiMAX (Thermo Fisher Scientific, Inc.) according to Lipofectamine RNAiMAX Reagent Protocol. For preparation of an ASO transfection solution, an RNAiMAX Transfection Reagent was mixed with 50 µL of an Opti-MEM solution so as to have a ratio of 2 µL, thereby preparing an RNAiMAX preparation solution. A preparation solution (ASO diluent) obtained by diluting ASO with Opti-MEM was mixed so as to be equal in volume to the RNAiMAX preparation solution, and 100 µL of the mixture was added to each well of a 24-well plate. Then, 500 µL of the cell suspension was seeded so that the human primary hepatic stellate cells were 2.5 × 10⁴ cells/well, and the cells were allowed to stand in a CO₂ incubator (final concentration of ASO: 100 nM). 48 hours after transfection, the medium was changed to D-MEM medium containing 0.2% FBS. 24 hours after medium change, human TGF-β1 protein (PeproTech, Inc.) was added so as to have a final concentration of 3 ng/mL. RNAs were extracted from the cells 48 hours after TGF-β1 stimulation using RNeasy 96 Kit (Qiagen N.V.). The RNAs thus obtained were subjected to a reverse transcription reaction using High Capacity cDNA Reverse Transcription Kit (Applied Biosystems(R)). The transcript amount of NDR2 was measured using NDR2 Taqman Gene Expression Assays (Hs00960027_m1, Thermo Fisher Scientific, Inc.), and the relative ratio of the transcript amount of GAPDH as an internal standard to the value measured by GAPDH Taqman Gene Expression Assays (Hs02786624_g1) was taken as the transcript amount of gene.

For the sequence of ASO, Antisense LNA GapmeR (SEQ ID NOs: 3, 4, 5, and 6) was used.

### <Antisense LNA GapmeR>

ASONDR2:5'-CAGTTGACTAGATAGG-3' (SEQ ID NO: 3)
ASONDR2:5'-GATCGACGTAACTTTT-3' (SEQ ID NO: 4)
ASONDR2:5'-TTAACCAGCCATGATA-3' (SEQ ID NO: 5)
ASONDR2:5'-CCATCTTCAGGAGTAT-3' (SEQ ID NO: 6)

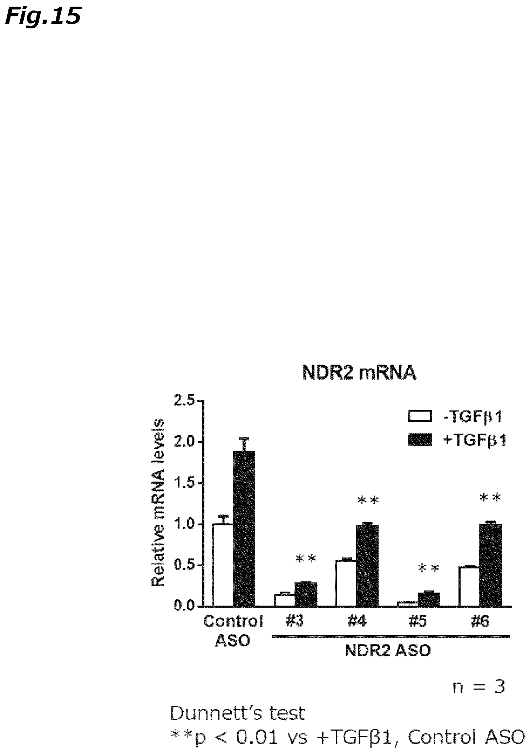

FIG. 15 illustrates the results of real-time PCR of NDR2 when Antisense LNA GapmeR for NDR2 was introduced into human hepatic stellate cells. The transcript amount of the NDR2 gene was suppressed by the introduction of NDR2 ASO. Therefore, it was shown that the expression of the target gene in the human hepatic stellate cells was efficiently suppressed by the NDR2 ASO used.

### [Example 8: Influence on Collagen Secretion by NDR2 ASO in Human Hepatic Stellate Cells]

In order to examine the possibility that NDR2 regulates collagen secretion in human primary hepatic stellate cells (Samsara Sciences, Inc.) isolated from human liver, the amount of collagen secreted in cells transfected with NDR2 ASO was analyzed.

To human primary hepatic stellate cells, ASO (Antisense LNA GapmeR, Qiagen N.V.) for human NDR2 was transfected using Lipofectamine RNAiMAX (Thermo Fisher Scientific, Inc.) according to Lipofectamine RNAiMAX Reagent Protocol. For preparation of an ASO transfection solution, an RNAiMAX Transfection Reagent was mixed with 50 µL of an Opti-MEM solution so as to have a ratio of 2 µL, thereby preparing an RNAiMAX preparation solution. A preparation solution (ASO diluent) obtained by diluting ASO with Opti-MEM was mixed so as to be equal in volume to the RNAiMAX preparation solution, and 100 µL of the mixture was added to each well of a 24-well plate. Then, 500 µL of the cell suspension was seeded so that the human primary hepatic stellate cells were 2.5 × 10⁴ cells/well, and the cells were allowed to stand in a CO₂ incubator (final concentration of ASO: 100 nM). 48 hours after transfection, the medium was changed to D-MEM medium containing 0.2% FBS. 24 hours after medium change, human TGF-β1 protein (PeproTech, Inc.) was added so as to have a final concentration of 3 ng/mL. 48 hours after TGF-β1 stimulation, 300 µL of the culture supernatant was added to and mixed with 1 mL of ice-cooled Sircol Dye Reagent (Biocolor Ltd) in a Protein LoBind tube (Eppendorf). The tube was attached to a small rotary incubator, mixing was performed at 25°C for 30 minutes, centrifugation (4°C, 12,000 x g, for 10 minutes) was performed, and then, the supernatant was removed. Then, 750 µL of a cooled Acid-Salt Wash Reagent (Biocolor Ltd) was added thereto, and after centrifugation (4°C, 12,000 x g, for 10 minutes), the supernatant was removed. Then, 250 µL of Sircol Collagen Assay ALKALI Reagent (Biocolor Ltd) was added, and mixing was performed. 200 µL of the mixed solution was transferred to a 96-well plate, and an absorbance at 555 nm was measured using an absorption microplate reader.

For the sequence of ASO, Antisense LNA GapmeR (SEQ ID NOs: 3, 4, 5, and 6) was used.

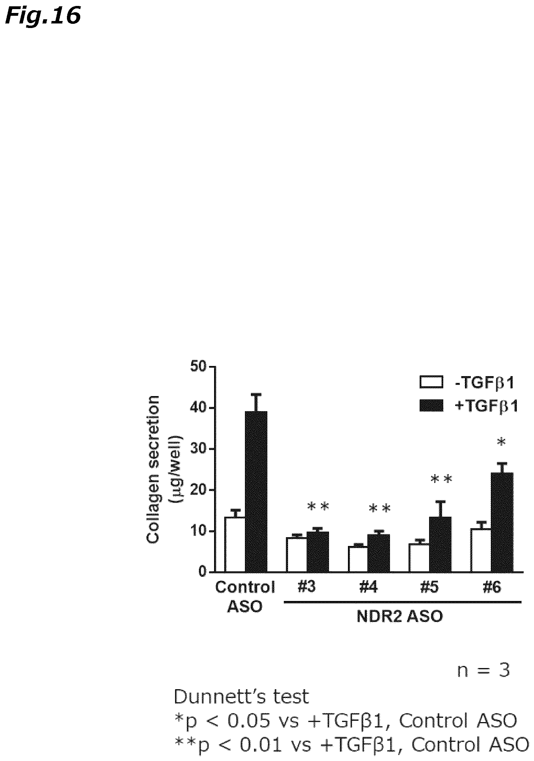

FIG. 16 illustrates the measurement results of collagen secretion when Antisense LNA GapmeR for NDR2 was introduced into human hepatic stellate cells. By using NDR2 ASO, the gene expression level of NDR2 was reduced, and the collagen secretion amount increased by TGF-β1 was reduced. Therefore, it was shown that collagen secretion was suppressed by NDR2 knockdown by NDR2 ASO in the human hepatic stellate cells.

### [Example 9: Knockdown by NDR2 siRNAs in Human Hepatic Stellate Cells]

The influence of siRNAs on the expression of NDR2 in human primary hepatic stellate cells (Samsara Sciences, Inc.) isolated from human liver was analyzed.

To human primary hepatic stellate cells, siRNAs for human NDR2 (ON-TARGET plus siRNA (Dharmacon Inc.) or Silencer Select siRNA (Thermo Fisher Scientific, Inc.)) were transfected using Lipofectamine RNAiMAX (Thermo Fisher Scientific, Inc.) according to Lipofectamine RNAiMAX Reagent Protocol. For preparation of an siRNA transfection solution, an RNAiMAX Transfection Reagent was mixed with 50 µL of an Opti-MEM solution so as to have a ratio of 1 µL, thereby preparing an RNAiMAX preparation solution. A preparation solution (siRNA diluent) obtained by diluting siRNAs with Opti-MEM was mixed so as to be equal in volume to the RNAiMAX preparation solution, and 100 µL of the mixture was added to each well of a 24-well plate. Then, 500 µL of a cell suspension was seeded so that the human primary hepatic stellate cells were 2.5 × 10⁴ cells/well, and the cells were allowed to stand in a CO₂ incubator (the final concentration of the ON-TARGET plus siRNA was 10 nM, and the final concentration of the Silencer Select siRNA was 3 nM). 48 hours after transfection, the medium was changed to D-MEM medium containing 0.2% FBS.

24 hours after medium change, human TGF-β1 protein (PeproTech, Inc.) was added so as to have a final concentration of 3 ng/mL. RNAs were extracted from the cells 48 hours after TGF-β1 stimulation using RNeasy 96 Kit (Qiagen N.V.). The RNAs thus obtained were subjected to a reverse transcription reaction using High Capacity cDNA Reverse Transcription Kit (Applied Biosystems(R)). The transcript amount of NDR2 was measured using NDR2 Taqman Gene Expression Assays (Hs00960027_m1, Thermo Fisher Scientific, Inc.), and the relative ratio of the transcript amount of GAPDH as an internal standard to the value measured by GAPDH Taqman Gene Expression Assays (Hs02786624_g1) was taken as the transcript amount of gene.

As the sequence of siRNA, ON-TARGET plus siRNA (SEQ ID NOs: 23 and 30), ON-TARGET plus siRNA (SEQ ID NOs: 24 and 31), and Silencer Select siRNA (SEQ ID NOs: 27 and 34) were used.

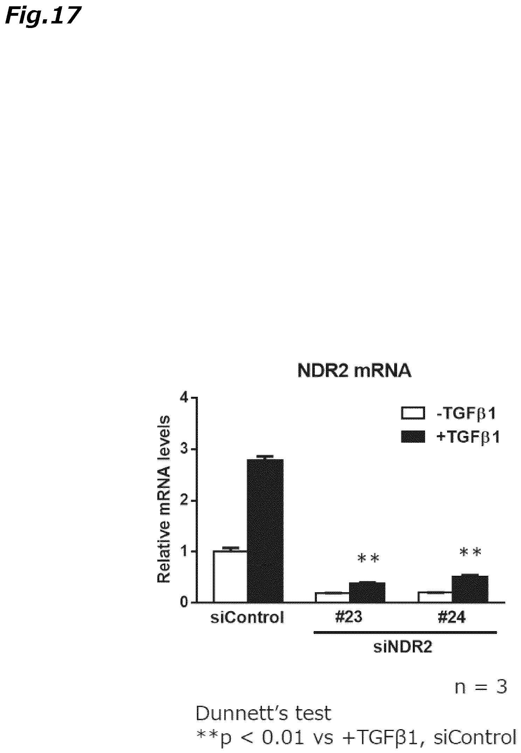

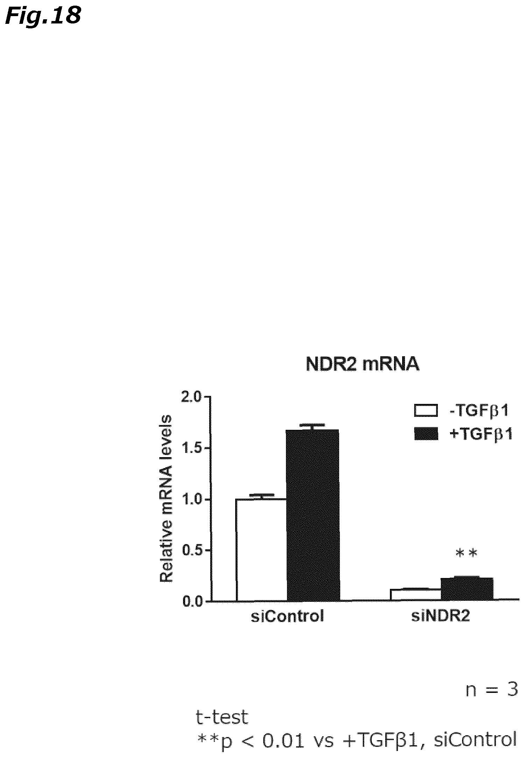

FIG. 17 illustrates the results of real-time PCR of NDR2 when ON-TARGET plus siRNA for NDR2 was introduced into human hepatic stellate cells. In addition, FIG. 18 illustrates the results of real-time PCR of NDR2 when Silencer Select siRNA was introduced into the human hepatic stellate cells. The transcript amount of the NDR2 gene was suppressed by the introduction of NDR2 siRNAs. Therefore, it was shown that the expression of the target gene in the human hepatic stellate cells was suppressed by the NDR2 siRNAs used.

### [Example 10: Influence of Knockdown by NDR2 siRNAs on Collagen Secretion in Human Hepatic Stellate Cells]

In order to examine the possibility that NDR2 regulates collagen secretion in human primary hepatic stellate cells (Samsara Sciences, Inc.) isolated from human liver, the amount of collagen secreted in cells transfected with NDR2 siRNAs was analyzed.

To human primary hepatic stellate cells, siRNAs for human NDR2 (ON-TARGET plus siRNA (Dharmacon Inc.) or Silencer Select siRNA (Thermo Fisher Scientific, Inc.)) were transfected using Lipofectamine RNAi MAX (Thermo Fisher Scientific, Inc.) according to Lipofectamine RNAiMAX Reagent Protocol. For preparation of an siRNA transfection solution, an RNAiMAX Transfection Reagent was mixed with 50 µL of an Opti-MEM solution so as to have a ratio of 1 µL, thereby preparing an RNAiMAX preparation solution. A preparation solution (siRNA diluent) obtained by diluting siRNAs with Opti-MEM was mixed so as to be equal in volume to the RNAiMAX preparation solution, and 100 µL of the mixture was added to each well of a 24-well plate. Then, 500 µL of a cell suspension was seeded so that the human primary hepatic stellate cells were 2.5 × 10⁴ cells/well, and the cells were allowed to stand in a CO₂ incubator (The final concentration of ON-TARGET plus siRNA was 10 nM and the final concentration of Silencer Select siRNA was 3 nM). 48 hours after transfection, the medium was changed to D-MEM medium containing 0.2% FBS.

24 hours after medium change, human TGF-β1 protein (PeproTech, Inc.) was added so as to have a final concentration of 3 ng/mL. 48 hours after TGF-β1 stimulation, 300 µL of the culture supernatant was added to and mixed with 1 mL of ice-cooled Sircol Dye Reagent (Biocolor Ltd) in a Protein LoBind tube (Eppendorf). The tube was attached to a small rotary incubator, mixing was performed at 25°C for 30 minutes, centrifugation (4°C, 12,000 x g, for 10 minutes) was performed, and then, the supernatant was removed. Then, 750 µL of a cooled Acid-Salt Wash Reagent (Biocolor Ltd) was added thereto, and after centrifugation (4°C, 12,000 x g, for 10 minutes), the supernatant was removed. Then, 250 µL of Sircol Collagen Assay ALKALI Reagent (Biocolor Ltd) was added, and mixing was performed. 200 µL of the mixed solution was transferred to a 96-well plate, and an absorbance at 555 nm was measured using an absorption microplate reader.

As the sequence of siRNA, ON-TARGET plus siRNA (SEQ ID NOs: 23 and 30), ON-TARGET plus siRNA (SEQ ID NOs: 24 and 31), and Silencer Select siRNA (SEQ ID NOs: 27 and 34) were used.

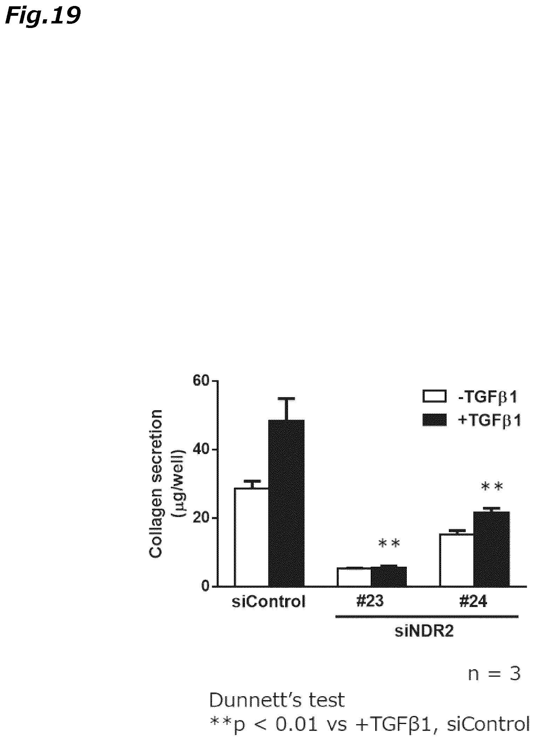

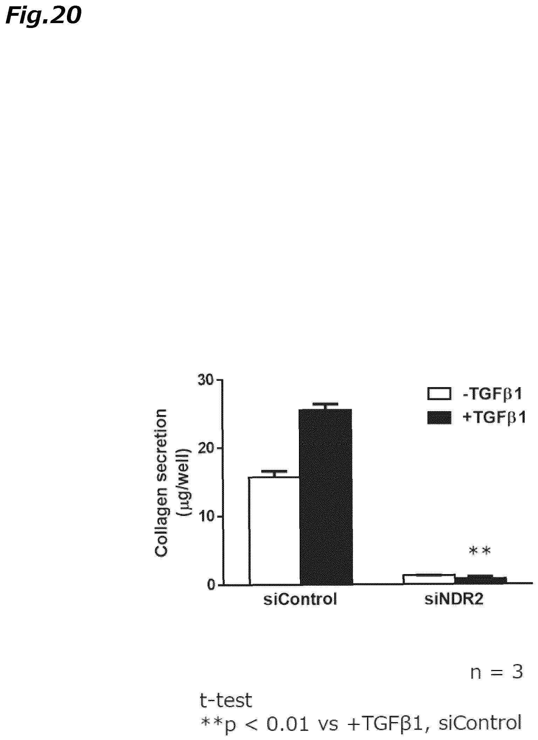

FIG. 19 illustrates the results of collagen secretion when ON-TARGET plus siRNA for NDR2 was introduced into the human hepatic stellate cells. In addition, FIG. 20 illustrates the results of collagen secretion when Silencer Select siRNA was introduced into the human hepatic stellate cells. The collagen secretion was suppressed by the introduction of NDR2 siRNAs. Therefore, it was shown that collagen secretion was suppressed by NDR2 knockdown by NDR2 siRNAs in the human hepatic stellate cells.

### [Example 11: Influence of Knockdown by NDR2 siRNAs on Collagen Secretion in Human Renal Fibroblasts]

The influence of siRNAs on the expression of NDR2 and the influence on collagen secretion in human cultured renal fibroblast HRF cells (Cell Biologics Inc.) were analyzed.

To HRF cells, siRNAs (ON-TARGET plus pool siRNA (Dharmacon Inc.) for human NDR2 were transfected using Lipofectamine RNAiMAX (Thermo Fisher Scientific, Inc.) according to Lipofectamine RNAiMAX Reagent Protocol. 500 µL of a cell suspension was seeded so that the HRF cells were 6.0 x 10⁴ cells/well on a 24-well plate, and the cells were allowed to stand in a CO₂ incubator for 24 hours. RNAiMAX Transfection Reagent Transfection was mixed with 25 µL of an Opti-MEM solution so as to have a ratio of 1 µL, thereby preparing an RNAiMAX preparation solution. A preparation solution (siRNA diluent) obtained by diluting siRNA with Opti-MEM was mixed so as to be equal in volume to the RNAiMAX preparation solution. To 50 µL of the mixed solution, a maintenance medium (basal medium containing 9% fetal bovine serum, 0.9% L-Glutamone, 0.09% FGF, and 0.09% Hydrocortisone) was added at a ratio of 450 µL, and 500 µL of the maintenance medium was added to each well of a 24-well plate (final concentration of siRNA: 30 nM). 24 hours after transfection, the medium was replaced with Complete Fibroblast Medium/w (Cell Biologics Inc.). 24 hours after medium change, human TGF-β1 protein (PeproTech, Inc.) was added so as to have a final concentration of 3 ng/mL.

RNAs were extracted from the cells 48 hours after TGF-β1 stimulation using RNeasy Mini Kit (Qiagen N.V.). The RNAs thus obtained were subjected to a reverse transcription reaction using High Capacity cDNA Reverse Transcription Kit (Applied Biosystems(R)). The transcript amount of NDR2 was measured using NDR2 Taqman Gene Expression Assays (Hs00960027_m1, Thermo Fisher Scientific, Inc.), and the relative ratio of the transcript amount of GAPDH as an internal standard to the value measured by GAPDH Taqman Gene Expression Assays (Hs02786624_g1) was taken as the transcript amount of gene.

In the collagen secretion measurement, 5% AnaeroPack Kenki and an O₂ meter were placed in a pouch bag under a low oxygen (1% O₂) condition, a 24-well plate was placed therein, and the pouch bag was allowed to stand in a CO₂ incubator. 48 hours after TGF-β1 stimulation, 350 µL of the culture supernatant was added to and mixed with 1 mL of ice-cooled Sircol Dye Reagent (Biocolor Ltd) in a Protein LoBind tube (Eppendorf). The tube was attached to a small rotary incubator, mixing was performed at 25°C for 30 minutes, centrifugation (4°C, 12,000 x g, for 10 minutes) was performed, and then, the supernatant was removed. Then, 750 µL of a cooled Acid-Salt Wash Reagent (Biocolor Ltd) was added thereto, and after centrifugation (4°C, 12,000 x g, for 10 minutes), the supernatant was removed. Then, 250 µL of Sircol Collagen Assay ALKALI Reagent (Biocolor Ltd) was added, and mixing was performed. 200 µL of the mixed solution was transferred to a 96-well plate, and an absorbance at 555 nm was measured using an absorption microplate reader.

As the sequence of siRNA, ON-TARGET plus pool siRNA (the mixture of SEQ ID NOs: 23 to 26 and SEQ ID NOs: 30 to 33 in equal amounts) was used.

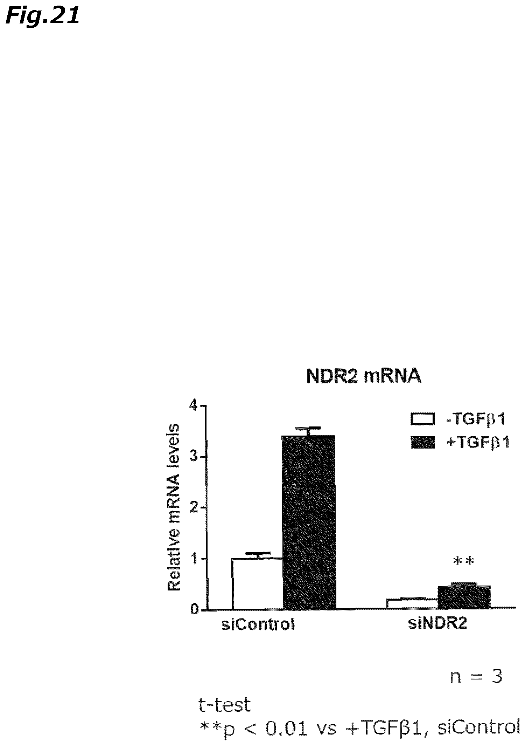

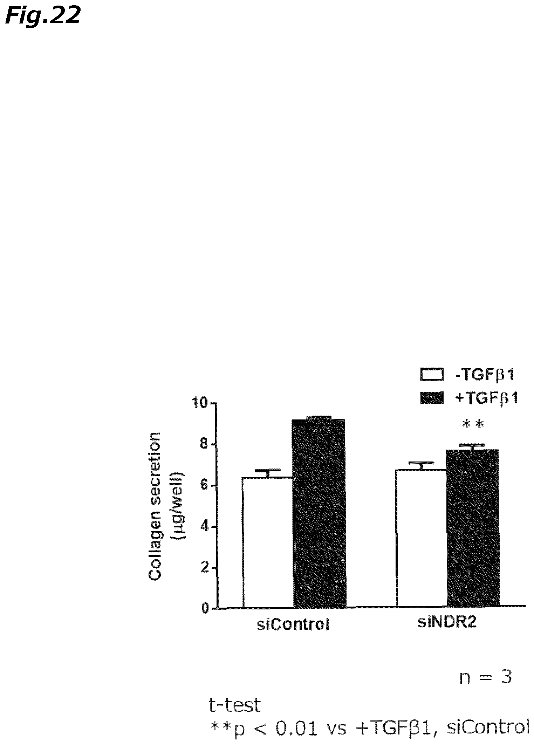

FIG. 21 illustrates the results of real-time PCR of NDR2 when ON-TARGET plus pool siRNA for NDR2 was introduced into human renal fibroblasts, and FIG. 22 illustrates the results of collagen secretion. The transcript amount of NDR2 gene in the human renal fibroblasts was suppressed by the introduction of NDR2 siRNAs. In addition, the collagen secretion was suppressed by the introduction of NDR2 siRNAs. Therefore, it was shown that collagen secretion was suppressed by NDR2 knockdown in the human renal fibroblasts.

### [Example 12: ASO Concentration-dependent NDR2 Knockdown in Mouse Pulmonary Fibroblasts]

The influence of ASO on the expression of NDR2 in cultured pulmonary fibroblasts MLg-2908 (ATCC) derived from a normal mouse was analyzed.

To MLg-2908 cells, ASO (Antisense LNA GapmeR, Qiagen N.V.) for mouse NDR2 was transfected using Lipofectamine RNAiMAX (Thermo Fisher Scientific, Inc.) according to Lipofectamine RNAiMAX Reagent Protocol. RNAiMAX Transfection Reagent Transfection was mixed with 50 µL of an Opti-MEM solution so as to have a ratio of 2 µL, thereby preparing an RNAiMAX preparation solution. A preparation solution (ASO diluent) obtained by diluting ASO with Opti-MEM was mixed so as to be equal in volume to the RNAiMAX preparation solution, and 100 µL of the mixture was added to each well of a 24-well plate. Then, 500 µL of the cell suspension was seeded so that the MLg-2908 cells were 4.0 × 10⁴ cells/well on a 24-well plate, and the cells were allowed to stand in a CO₂ incubator (prepared so that the final concentration of ASO was a target concentration). 24 hours after transfection, the medium was changed to MEM medium containing 0.2% FBS. 24 hours after medium change, human TGF-β1 protein (PeproTech, Inc.) was added so as to have a final concentration of 3 ng/mL. RNAs were extracted from the cells 18 hours after TGF-β1 stimulation using RNeasy 96 Kit (Qiagen N.V.). The RNAs thus obtained were subjected to a reverse transcription reaction using High Capacity cDNA Reverse Transcription Kit (Applied Biosystems(R)). The transcript amount of NDR2 was measured using NDR2 Taqman Gene Expression Assays (Mm00730941_s1, Thermo Fisher Scientific, Inc.), and the relative ratio of the transcript amount of 18S as an internal standard to the value measured by 18S Taqman Gene Expression Assays (Hs99999901_s1) was taken as the transcript amount of gene.

For the sequence of ASO, Antisense LNA GapmeR was used. (SEQ ID NOs: 15 and 17)

### <Antisense LNA GapmeR>

ASONDR2:5'-AGTTGATTAAGATAGG-3' (SEQ ID NO: 15)
ASONDR2:5'-TTACCTGGATTGACAA-3' (SEQ ID NO: 17)

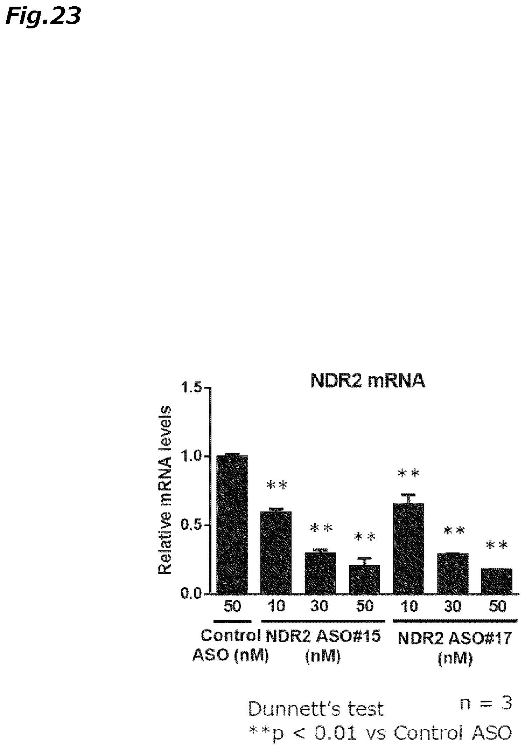

FIG. 23 illustrates the results of real-time PCR of NDR2 when Antisense LNA GapmeR for NDR2 was introduced into the MLg-2908 cells. The transcript amount of NDR2 gene in the mouse pulmonary fibroblasts was suppressed by the introduction of NDR2 ASO in an ASO concentration-dependent manner.

### [Example 13: Influence on Procollagen Secretion by NDR2 ASO in Mouse Pulmonary Fibroblasts]

In order to examine the possibility that NDR2 regulates collagen secretion in cultured pulmonary fibroblasts MLg-2908 (ATCC) derived from a normal mouse, the amount of procollagen secreted in cells transfected with NDR2 ASO was analyzed.

To MLg-2908 cells, ASO (Antisense LNA GapmeR, Qiagen N.V.) for mouse NDR2 was transfected using Lipofectamine RNAiMAX (Thermo Fisher Scientific, Inc.). RNAiMAX Transfection Reagent Transfection was mixed with 50 µL of an Opti-MEM solution so as to have a ratio of 2 µL, thereby preparing an RNAiMAX preparation solution. A preparation solution (ASO diluent) obtained by diluting ASO with Opti-MEM was mixed so as to be equal in volume to the RNAiMAX preparation solution, and 100 µL of the mixture was added to each well of a 24-well plate. Then, 500 µL of the cell suspension was seeded so that the MLg-2908 cells were 4.0 × 10⁴ cells/well on a 24-well plate, and the cells were allowed to stand in a CO₂ incubator (prepared so that the final concentration of ASO was a target concentration). 24 hours after transfection, the medium was changed to MEM medium containing 0.2% FBS. 24 hours after medium change, human TGF-β1 protein (PeproTech, Inc.) was added so as to have a final concentration of 3 ng/mL. The culture supernatant 48 hours after TGF-β1 stimulation was treated according to the method of Mouse Pro-Collagen I alpha 1 SimleStep ELISA Kit (Abcam plc.), and the absorbance of the sample obtained at 450 nm was measured using an absorption microplate reader.

For the sequence of ASO, Antisense LNA GapmeR was used. (SEQ ID NOs: 15 and 17)

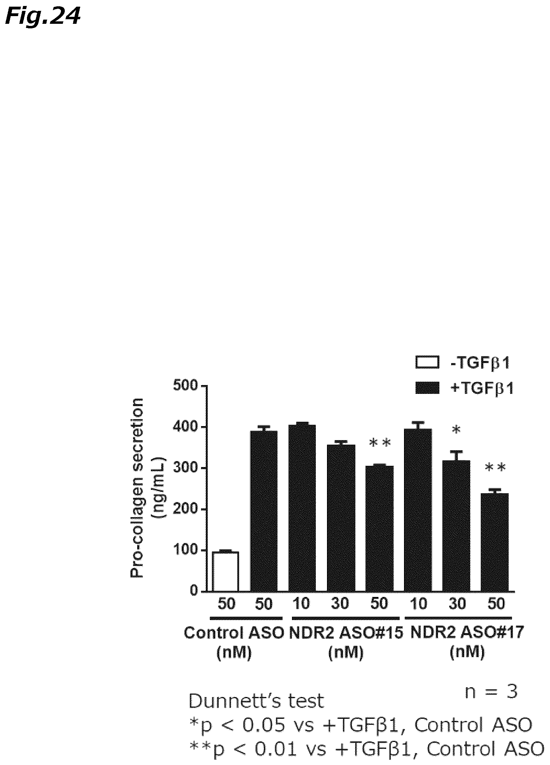

FIG. 24 illustrates the results of procollagen secretion when Antisense LNA GapmeR against NDR2 was introduced into the MLg-2908 cells. By using ASO for NDR2, the gene expression level of NDR2 was reduced, and the amount of procollagen secreted, which is increased by TGF-β, was reduced in an ASO concentration-dependent manner. Therefore, it was shown that the procollagen secretion in the mouse pulmonary fibroblasts was suppressed by NDR2 knockdown.

### [Example 14: NDR2 Knockdown Using siRNAs in Mouse Hepatic Stellate Cells]

The influence of siRNAs on the expression of NDR2 in mouse primary hepatic stellate cells (ScienCell) isolated from mouse liver was analyzed.

To mouse primary hepatic stellate cells, siRNAs for mouse NDR2 (Ambion In Vivo siRNA, Thermo Fisher Scientific, Inc.) were transfected using Lipofectamine RNAiMAX (Thermo Fisher Scientific, Inc.) according to Lipofectamine RNAiMAX Reagent Protocol. RNAiMAX Transfection Reagent Transfection was mixed with 50 µL of an Opti-MEM solution so as to have a ratio of 2 µL, thereby preparing an RNAiMAX preparation solution. A preparation solution (siRNA diluent) obtained by diluting siRNAs with Opti-MEM was mixed so as to be equal in volume to the RNAiMAX preparation solution, and 100 µL of the mixture was added to each well of a 24-well plate. 500 µL of a cell suspension was seeded so that the mouse primary hepatic stellate cells were 2.8 × 10⁴ cells/well on a 24-well plate, and the cells were allowed to stand in a CO₂ incubator for 24 hours (final concentration of siRNA: 10 nM). 24 hours after transfection, the medium was replaced with Stellate Cell Medium (Sciencell) containing 0.2% FBS. 24 hours after medium change, human TGF-β1 protein (PeproTech, Inc.) was added so as to have a final concentration of 10 ng/mL. RNAs were extracted from the cells 24 hours after TGF-β1 stimulation using RNeasy 96 Kit (Qiagen N.V.). The RNAs thus obtained were subjected to a reverse transcription reaction using High Capacity cDNA Reverse Transcription Kit (Applied Biosystems(R)). The transcript amount of NDR2 was measured using NDR2 Taqman Gene Expression Assays (Mm00730941_s1, Thermo Fisher Scientific, Inc.), and the relative ratio of the transcript amount of 18S as an internal standard to the value measured by 18S Taqman Gene Expression Assays (Hs99999901_s1) was taken as the transcript amount of gene.

As the sequence of siRNA, Ambion In Vivo siRNA (Thermo Fisher Scientific, Inc.) was used (SEQ ID NOs: 28 and 35 and SEQ ID NOs: 29 and 36).

### <Ambion In Vivo siRNA>

siNDR2:Sense:5'-CAGACAACCUUUUACUGGAtt-3' (SEQ ID NO: 35)
Antisense:5'-UCCAGUAAAAGGUUGUCUGgt-3' (SEQ ID NO: 28)
siNDR2:Sense:5'-CCAUACCCGGGAAAGAGUAtt-3' (SEQ ID NO: 36)
Antisense:5'-UACUCUUUCCCGGGUAUGGtt-3' (SEQ ID NO: 29)

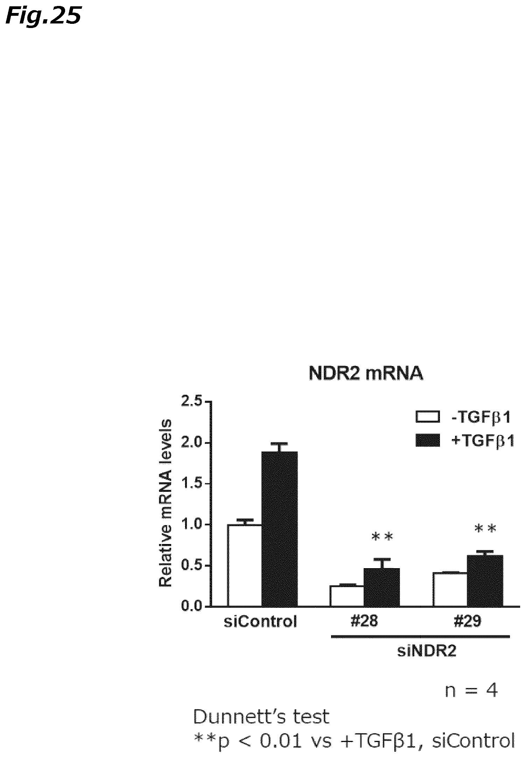

FIG. 25 illustrates the results of real-time PCR of NDR2 when Ambion In Vivo siRNA for NDR2 was introduced into the mouse hepatic stellate cells. The transcript amount of NDR2 gene in the mouse hepatic stellate cells was suppressed by the introduction of NDR2 siRNAs.

### [Example 15: Influence of NDR2 Knockdown on Collagen Secretion in Mouse Hepatic Stellate Cells]

In order to examine the possibility that NDR2 regulates collagen secretion in mouse hepatic stellate cells, the amount of secreted collagen was analyzed in cells transfected with NDR2 siRNAs (Ambion In Vivo siRNA (Thermo Fisher Scientific, Inc.)).

To mouse primary hepatic stellate cells (ScienCell) isolated from mouse liver, siRNAs for mouse NDR2 (Ambion In Vivo siRNA, Thermo Fisher Scientific, Inc.) were transfected using Lipofectamine RNAiMAX (Thermo Fisher Scientific, Inc.) according to Lipofectamine RNAiMAX Reagent Protocol. RNAiMAX Transfection Reagent Transfection was mixed with 50 µL of an Opti-MEM solution so as to have a ratio of 2 µL, thereby preparing an RNAiMAX preparation solution. A nucleic acid diluted solution obtained by diluting siRNA with Opti-MEM was mixed so as to be equal in volume to the RNAiMAX preparation solution, and 100 µL of the mixture was added to each well of a 24-well plate. Then, 500 µL of a cell suspension was seeded so that the mouse primary hepatic stellate cells were 2.8 × 10⁴ cells/well on a 24-well plate, and the cells were allowed to stand in a CO₂ incubator for 24 hours (final concentration of siRNA: 10 nM). 24 hours after transfection, the medium was replaced with Stellate Cell Medium (Sciencell) containing 0.2% FBS. 24 hours after medium change, human TGF-β1 protein (PeproTech, Inc.) was added so as to have a final concentration of 10 ng/mL. 48 hours after TGF-β1 stimulation, 400 µL of the culture supernatant was added to and mixed with 1 mL of ice-cooled Sircol Dye Reagent (Biocolor Ltd) in a Protein LoBind tube (Eppendorf). The tube was attached to a small rotary incubator, mixing was performed at 25°C for 30 minutes, centrifugation (4°C, 12,000 x g, for 10 minutes) was performed, and then, the supernatant was removed. Then, 750 µL of a cooled Acid-Salt Wash Reagent (Biocolor Ltd) was added thereto, and after centrifugation (4°C, 12,000 x g, for 10 minutes), the supernatant was removed. Then, 250 µL of Sircol Collagen Assay ALKALI Reagent (Biocolor Ltd) was added, and mixing was performed. 200 µL of the mixed solution was transferred to a 96-well plate, and an absorbance at 555 nm was measured using an absorption microplate reader.

As the sequence of siRNA, Ambion In Vivo siRNA (Thermo Fisher Scientific, Inc.) was used (SEQ ID NOs: 28 and 35 and SEQ ID NOs: 29 and 36).

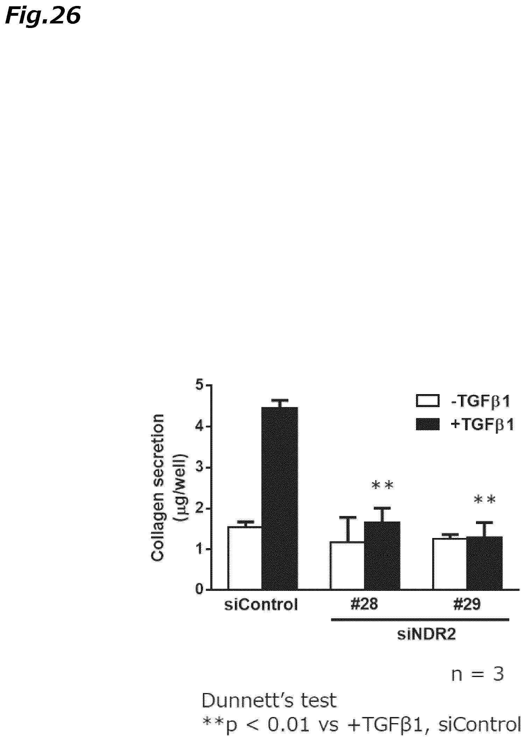

FIG. 26 illustrates the results of collagen secretion when Ambion In Vivo siRNA against NDR2 was introduced into the mouse hepatic stellate cells. By using siRNA for NDR2, the gene expression level of NDR2 was reduced, and the collagen secretion amount increased by TGF-β1 was reduced. Therefore, it was shown that the collagen secretion in the mouse hepatic stellate cells was suppressed by NDR2 knockdown.

### [Example 16: NDR2 Knockdown Using ASO in Mouse Lung]

The influence of NDR2 ASO on the transcript amount of the target gene in the mouse lung was analyzed.

Grouping of 9-weeks-old male C57BL/6J mice (Charles River Laboratories Japan, Inc.) was performed with the body weight on the day before the initial administration of NDR2 ASO, and the design of the groups was as a D-PBS administration group (n = 6), a single dose nucleic acid (QW) administration group (n = 6), and a nucleic acid three times a week (TIW) administration group (n = 6). NDR2 ASO was used to prepare 10 mg/mL of a nucleic acid stock solution using ultrapure water. In the QW administration group, a nucleic acid administration solution containing NDR2 ASO prepared using D-PBS (-) (Nacalai Tesque Inc.) as an administration solvent was oropharyngeal aspirated to the nucleic acid administration group so as to have a body weight of 1, 3 or 10 mg/kg (mpk), and the solvent administration group was oropharyngeal aspirated with an equal amount of the administration solvent to the nucleic acid administration group on day 0. In the TIW administration group, a nucleic acid administration solution containing NDR2 ASO prepared using D-PBS (-) (Nacalai Tesque Inc.) as an administration solvent was oropharyngeal aspirated on day 0, 2, and 4 so as to have a body weight of 1 mg/kg. Gene expression analysis was performed 72 hours after final administration.

A lung collected 72 hours after final administration was crushed under liquid nitrogen using a freeze-crush SK mill, and RNAs were extracted using a QIAZOL Lysis reagent (Qiagen N.V.). Subsequently, RNAs were purified using RNeasy mini Kit (Qiagen N.V.), and a reverse transcription reaction was performed using High Capacity cDNA Reverse Transcription Kit (Applied Biosystems(R)). The transcript amount of NDR2 was measured using NDR2 Taqman Gene Expression Assays (Mm00730941_s1, Thermo Fisher Scientific, Inc.), and the relative ratio of the transcript amount of 18S as an internal standard to the value measured by 18S Taqman Gene Expression Assays (Hs99999901_s1) was taken as the transcript amount of gene.

For the sequence of ASO, Antisense LNA GapmeR (SEQ ID NO: 14) was used.

### <Antisense LNA GapmeR>

ASONDR2:5'-TATGCCCAGTTGATTA-3' (SEQ ID NO: 14)

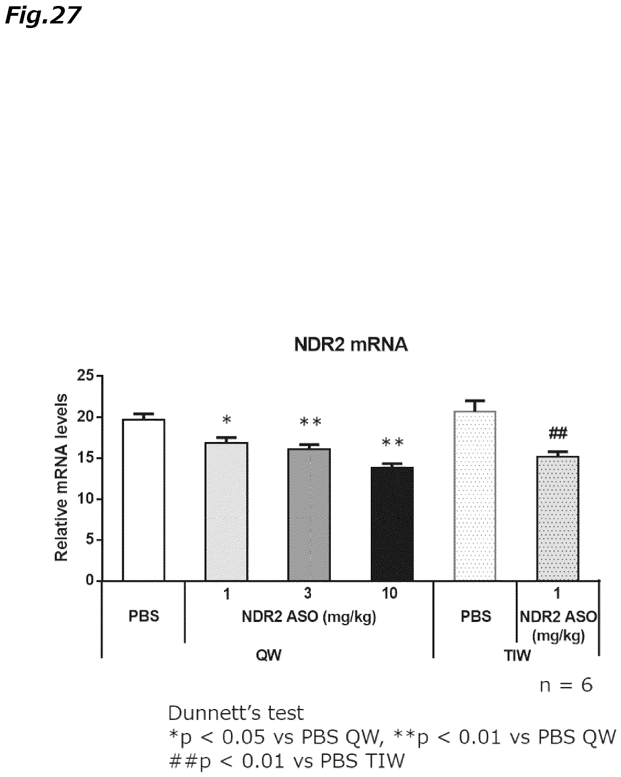

FIG. 27 illustrates the NDR2 gene transcript amount in the lung when Antisense LNA GapmeR for NDR2 was administered to normal mice. By the administration of NDR2 ASO, the transcript amount of the NDR2 gene was reduced. It was shown that the expression of the target gene in the lung was efficiently suppressed by the NDR2 Antisense LNA GapmeR used.

### [Example 17: NDR2 Knockdown using ASO in Mouse Liver]

The influence of NDR2 ASO on the transcript amount of the target gene in the mouse liver was analyzed.

Grouping of 9-weeks-old male C57BL/6J mice (Charles River Laboratories Japan, Inc.) was performed by stratified randomization of EXSUS using the body weights of the mice measured on the day of the initial administration of NDR2 ASO (n = 3 in each group). NDR2 ASO was used to prepare 10 mg/mL of a nucleic acid stock solution using ultrapure water. A nucleic acid administration solution containing NDR2 ASO prepared using D-PBS (-) (Nacalai Tesque Inc.) as an administration solvent was subcutaneously administered (s.c. administration) or administered via tail vein (i.v. administration) to the nucleic acid administration group so as to have a body weight of 10 or 30 mg/kg. In the solvent administration group, the same amount of administration solvent as that in the nucleic acid administration group was subcutaneously administered or administered via tail vein. Gene expression analysis was performed 48 hours after administration.

A liver collected 48 hours after administration was crushed under liquid nitrogen using a freeze-crush SK mill, and RNAs were extracted using a QIAZOL Lysis reagent (Qiagen N.V.). Subsequently, RNAs were purified using RNeasy mini Kit (Qiagen N.V.), and a reverse transcription reaction was performed using High Capacity cDNA Reverse Transcription Kit (Applied Biosystems(R)). The transcript amount of NDR2 was measured using NDR2 Taqman Gene Expression Assays (Mm00730941_s1, Thermo Fisher Scientific, Inc.), and the relative ratio of the transcript amount of 18S as an internal standard to the value measured by 18S Taqman Gene Expression Assays (Hs9999901_s1) was taken as the transcript amount of gene.

For the sequence of ASO, Antisense LNA GapmeR (SEQ ID NO: 14) was used.

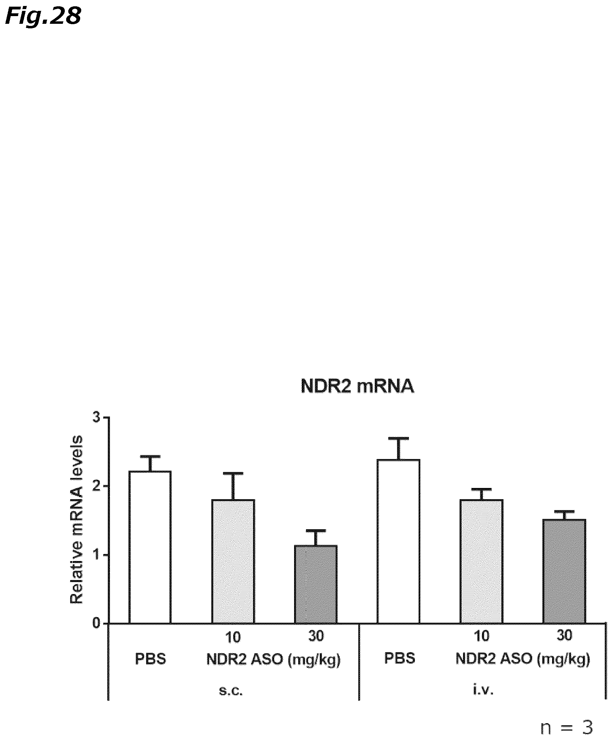

FIG. 28 illustrates the NDR2 gene transcript amount in the liver when NDR2 ASO was subcutaneously administered or intravenously administered to the tail. By the administration of NDR2 ASO, the transcript amount of the NDR2 gene was reduced. This showed that the expression of the target gene in the liver in subcutaneous administration or tail vein administration was efficiently suppressed by the NDR2 Antisense LNA GapmeR used.

### [Example 18: Influence of NDR2 Knockdown on Analogous Genes in Mouse Liver]

The influence of NDR2 siRNAs on the transcript amount of the analogous gene NDR1 gene in the normal mouse liver was analyzed.

Grouping of 8-weeks-old male C57BL/6J mice (Charles River Laboratories Japan, Inc.) was performed with the body weight on the day before the administration of NDR2 siRNA, and the design of the groups was as a PBS administration group (n = 3) or a nucleic acid administration group (n = 3). Using Ambion In Vivo siRNA (Thermo Fisher Scientific, Inc.) as NDR2 siRNA and Ambion In Vivo Negative control siRNA (Thermo Fisher Scientific, Inc.) as control siRNA, 0.2 or 0.3 mg/mL of a nucleic acid administration solution was prepared according to the product protocol of invivofectamine 3.0 using invivofectamine 3.0 Reagent (Thermo Fisher Scientific, Inc.). A nucleic acid administration solution containing Ambion In Vivo siRNA was administered via tail vein to the nucleic acid administration group so as to have a body weight of 2 or 3 mg/kg, and PBS (Thermo Fisher Scientific, Inc.) in an amount equivalent to that in the nucleic acid administration group was administered via tail vein to the solvent administration group.

A liver collected 72 hours after administration was crushed under liquid nitrogen using a freeze-crush SK mill, and RNAs were extracted using a QIAZOL Lysis reagent (Qiagen N.V.). Subsequently, RNAs were purified using RNeasy mini Kit (Qiagen N.V.), and a reverse transcription reaction was performed using High Capacity cDNA Reverse Transcription Kit (Applied Biosystems(R)). As for the transcript amount of the NDR2 gene and the transcript amount of the NDR1 gene, the relative ratios of the measured value in NDR2 Taqman Gene Expression Assays (Mm00730941_s1, Thermo Fisher Scientific, Inc.) and the measured value in NDR1 Taqman Gene Expression Assays (Mm00506497_m1, Thermo Fisher Scientific, Inc.) to the measured value in 18S Taqman Gene Expression Assays (Hs99999901_s1) were defined as the transcript amounts of the genes, respectively.

As the sequence of siRNA, Ambion In Vivo siRNA (SEQ ID NOs: 28 and 35) was used.

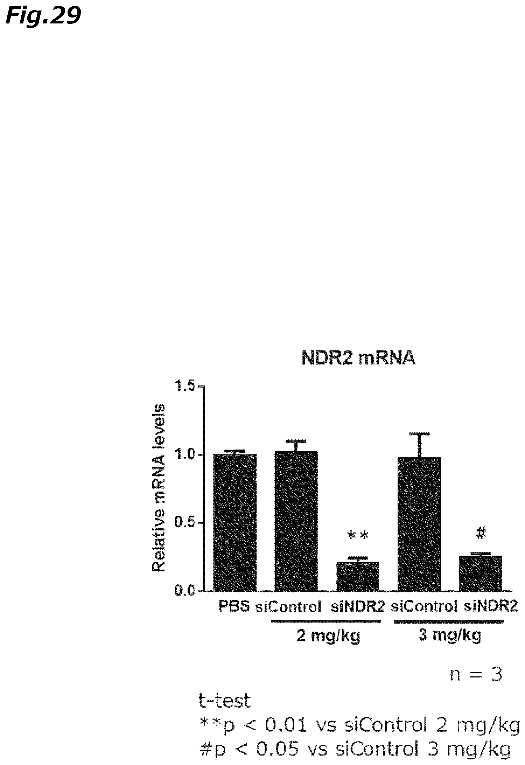

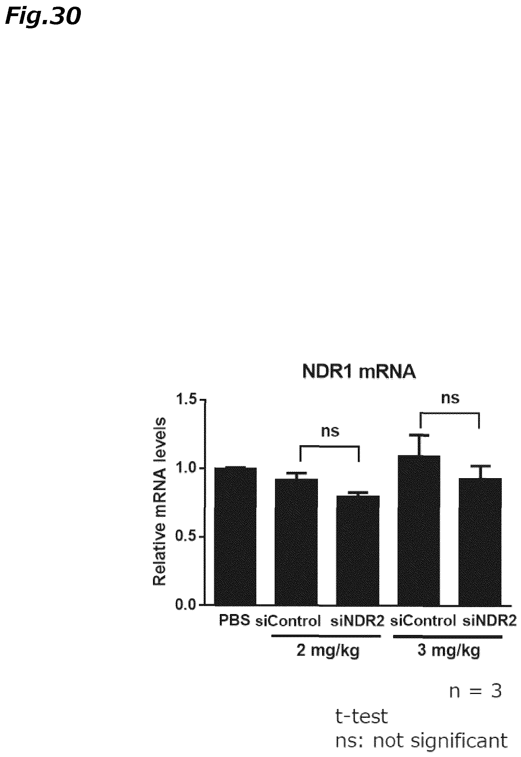

FIG. 29 illustrates the NDR2 gene transcript amount when the NDR2 siRNAs were administered to the normal mice, and FIG. 30 illustrates the NDR1 gene transcript amount when the NDR2 siRNAs were administered. By the administration of the NDR2 siRNA, the transcript amount of the NDR2 gene in the liver was reduced, but there was no change in the transcript amount of the NDR1 gene. This showed that the used NDR2 Ambion In Vivo siRNA did not affect the expression of the NDR1 gene, which is an analogous gene.

### [Example 19: Influence of NDR2 Knockdown on Drug-metabolizing Enzyme in Normal Mouse]

In order to confirm the influence of the NDR2 knockdown on a drug metabolizing enzyme, the influence on the drug metabolizing enzyme CYP2E1 (Cytochrome P450 Family 2 Subfamily E Member 1) protein when NDR2 siRNAs were administered via tail vein to a normal mouse was analyzed.

Grouping of 8-weeks-old male C57BL/6J mice (Charles River Laboratories Japan, Inc.) was performed with the body weight on the day before the administration of NDR2 siRNA, and the design of the groups was as a PBS administration group (n = 3) or a nucleic acid administration group (n = 3). Using Ambion In Vivo siRNA (Thermo Fisher Scientific, Inc.) as NDR2 siRNA and Ambion In Vivo Negative control siRNA (Thermo Fisher Scientific, Inc.) as control siRNA, 0.2 or 0.3 mg/mL of a nucleic acid administration solution was prepared according to the product protocol of invivofectamine 3.0 using invivofectamine 3.0 Reagent (Thermo Fisher Scientific, Inc.). A nucleic acid administration solution containing Ambion In Vivo siRNA or Ambion In Vivo Negative control siRNA was administered via tail vein to the nucleic acid administration group so as to have a body weight of 2 or 3 mg/kg, and PBS (Thermo Fisher Scientific, Inc.) in an amount equivalent to that in the nucleic acid administration group was administered via tail vein to the solvent administration group.

A liver collected 72 hours after administration was frozen and then crushed using a freeze-crush SK mill. A dissolution buffer (150 mM NaCl, 1% NP40, 0.1% SDS, 50 mM Tris-HCl pH 7.5, 1 mM EDTA, 1 mM Benzylsulfonyl fluoride, 2% protease inhibitor cocktail, 1% phosphatase inhibitor cocktail) was added to the liver crushed product, and an organ extract was prepared using a handy ultrasonic generator. β-Mercaptoethanol and Bromophenol blue were added to the supernatant obtained by centrifuging the organ extract at final concentrations of 5% and 0.025%, respectively. Thereafter, heating was performed at 95°C for 4 minutes to obtain a sample. SDS-PAGE was performed using the sample thus obtained, and proteins contained in the sample were separated by size. Thereafter, the separated protein was transferred to a PVDF membrane, and Western blotting was performed with an anti-CYP2E1 antibody (Abcam plc.) and an anti-Vinculin antibody (Sigma-Aldrich Co. LLC.).

As the sequence of siRNA, Ambion In Vivo siRNA (SEQ ID NOs: 28 and 35) was used.

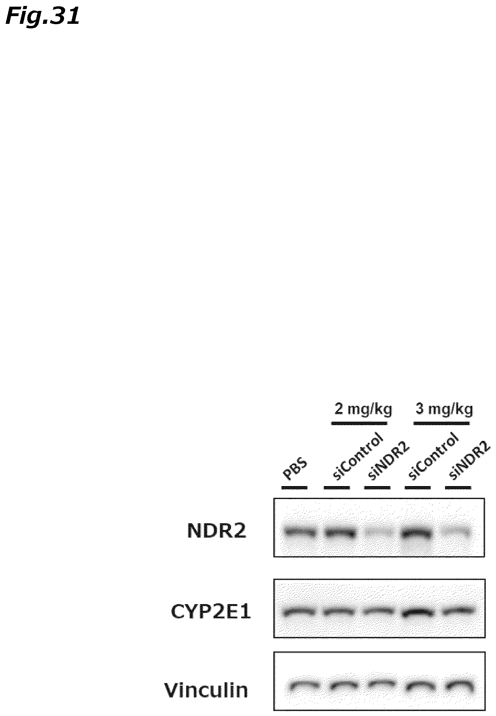

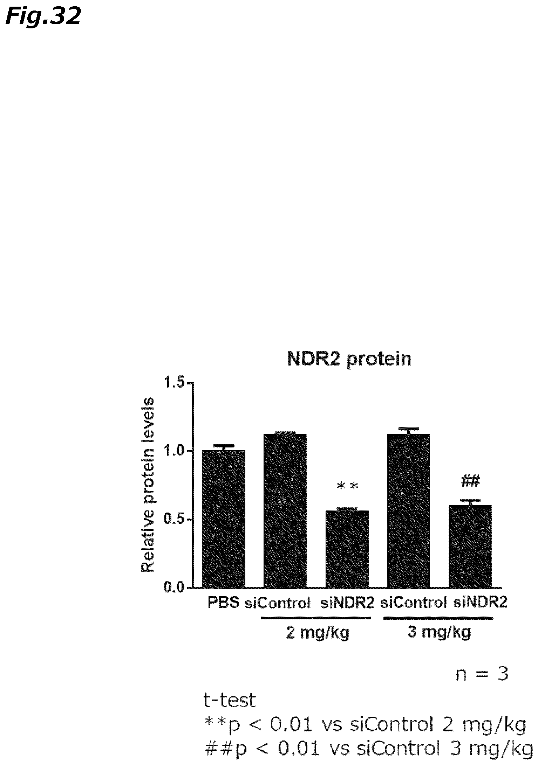

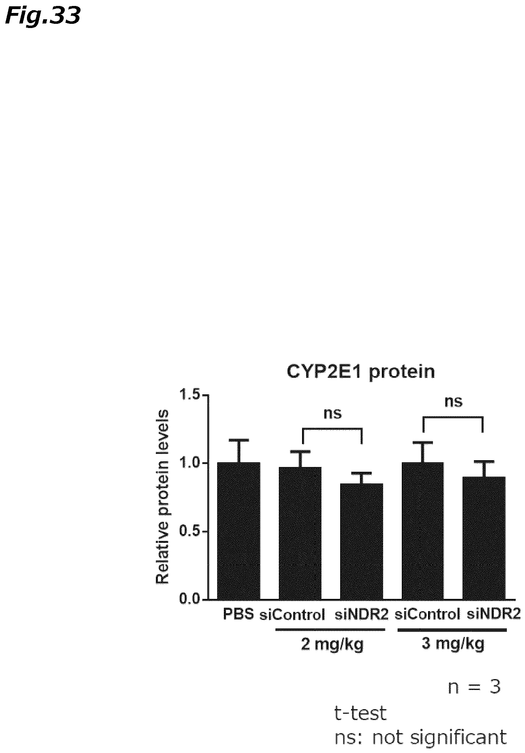

FIG. 31 illustrates the results of Western blotting of NDR2 protein and CYP2E1 protein when NDR2 was knocked down. In addition, the value of NDR2 protein corrected by Vinculin is graphed in FIG. 32, and the value of CYP2E1 protein corrected by Vinculin is graphed in FIG. 33. By using siRNA of NDR2, the NDR2 protein was reduced, but the amount of CYP2E1 protein was not changed. Therefore, it was shown that NDR2 knockdown does not affect CYP2E1 protein in the normal mice.

### [Example 20: Influence of NDR2 siRNAs on Fibrosis-associated Genes in Carbon Tetrachloride (CCl₄)-Induced Hepatic Fibrogenesis Model]

In order to confirm that NDR2 knockdown is effective for fibrosis, NDR2 siRNAs were intravenously administered to carbon tetrachloride (CCl₄)-induced hepatic fibrogenesis model mice (hereinafter, referred to as CCl₄ model mice), and the effect on a transcript amount of fibrosis-associated genes was analyzed.

To 8-weeks-old male C57BL/6J mice (Charles River Laboratories Japan, Inc.), an olive oil solution containing 12.5 v/v% CCl₄ (FUJIFILM Wako Pure Chemical Corporation) was intraperitoneally administered at 10 mL/kg by body weight on day 3 and 6 when the day on which the nucleic acid was first administered was day 0, thereby creating liver fibrosis model mice. Grouping was performed with body weight on the day before the initial administration of NDR2 siRNAs, and the design of the groups was as a PBS administration group not receiving CCl₄ (n = 3), a PBS administration group receiving CCl₄ (n =3), and a nucleic acid administration group receiving CCl₄ (n = 3). Using Ambion In Vivo siRNA (Thermo Fisher Scientific, Inc.) as NDR2 siRNA and Ambion In Vivo Negative control siRNA (Thermo Fisher Scientific, Inc.) as control siRNA, 0.2 mg/mL of a nucleic acid administration solution was prepared according to the product protocol of invivofectamine 3.0 using invivofectamine 3.0 Reagent (Thermo Fisher Scientific, Inc.). A nucleic acid administration solution containing Ambion In Vivo siRNA or Ambion In Vivo Negative control siRNA was administered via tail vein to the nucleic acid administration group so as to have a body weight of 2 mg/kg, and PBS (Thermo Fisher Scientific, Inc.) in an amount equivalent to that in the nucleic acid administration group was administered via tail vein to the solvent administration group on day 0 and 4. Evaluation of fibrosis was performed on day 8.

RNAs were extracted from the liver collected on day 8 using QIAZOL Lysis reagent (Qiagen N.V.). Subsequently, RNAs were purified using RNeasy mini Kit (Qiagen N.V.), and a reverse transcription reaction was performed using High Capacity cDNA Reverse Transcription Kit (Applied Biosystems(R)), thereby obtaining cDNAs. The cDNAs thus obtained were subjected to real-time PCR using TaqMan Fast Universal PCR master mix and Applied Biosystems 7500 Fast Real-Time PCR Systems (Applied Biosystems(R)), and the influence of NDR2 knockdown on the transcript amount of each gene was examined. The transcript amounts of NDR2 Taqman Probe (Mm00491127_m1, Thermo Fisher Scientific, Inc.) and CYR61 Taqman Probe (Mm00487498_m1, Thermo Fisher Scientific, Inc.), CTGF Taqman Probe (Mm01192933_g1, Thermo Fisher Scientific, Inc.), TNF Taqman Probe (Mm00443258_m1, Thermo Fisher Scientific, Inc.), CCL2 Taqman Probe (Mm00441242_m1, Thermo Fisher Scientific, Inc.), COL1A1 Taqman Probe (Mm801666_g1, Thermo Fisher Scientific, Inc.), and TGF-β1 Taqman Probe(Mm01178820_m1, Thermo Fisher Scientific, Inc.) as fibrosis-associated genes were measured using TaqMan Gene Expression Assays, and the relative ratio to the transcript amount of 18 S Taqman Probe (Hs99999901_s1) as an internal standard was defined as the transcript amount of each gene.

As the sequence of siRNA, Ambion In Vivo siRNA (SEQ ID NOs: 28 and 35) was used.

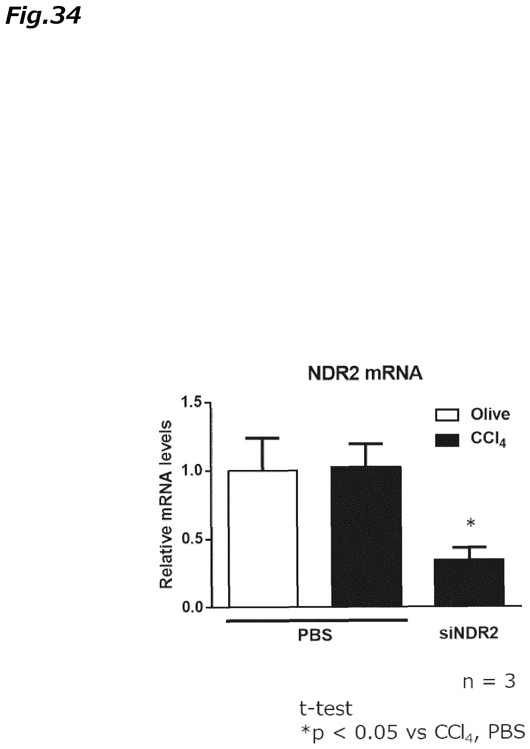

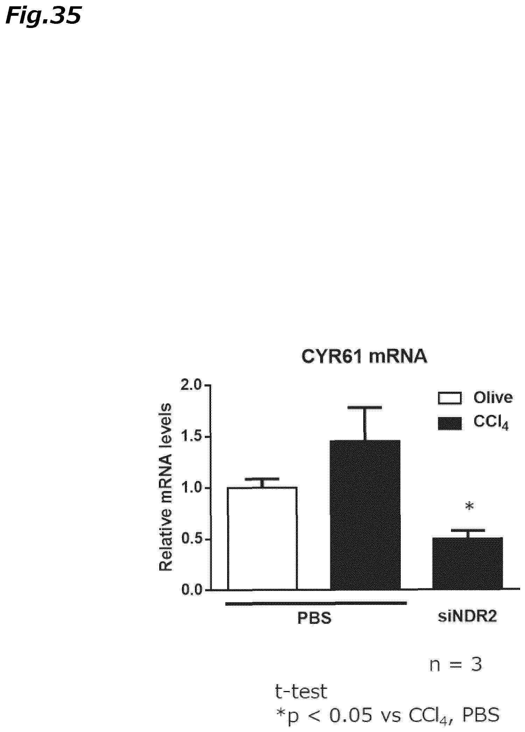

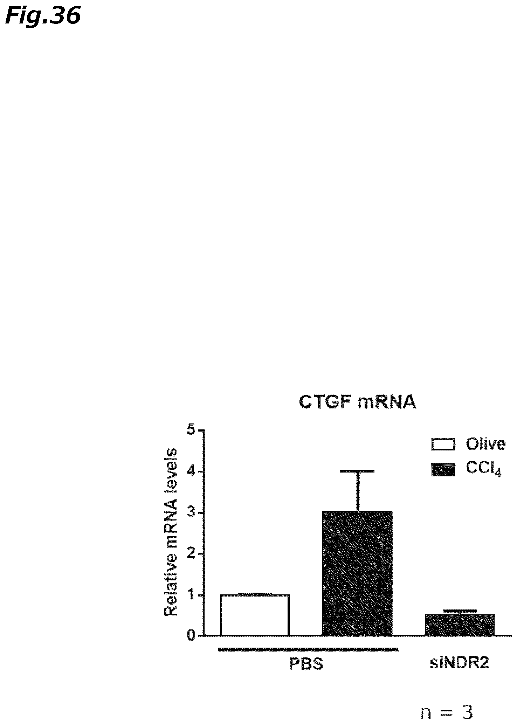

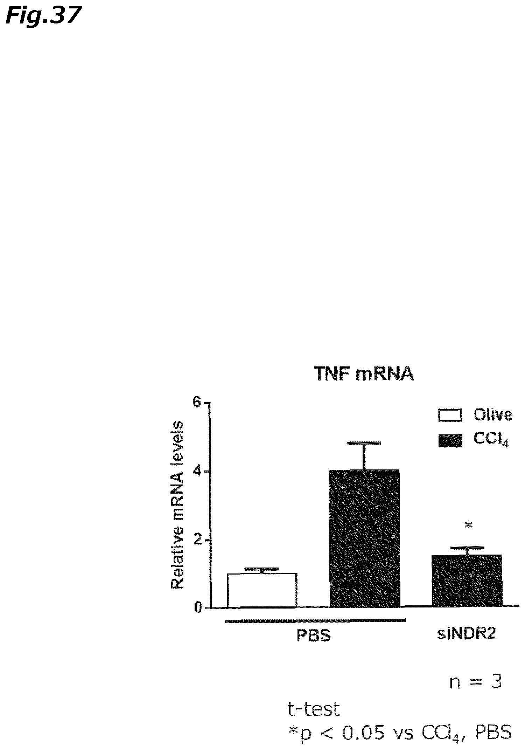

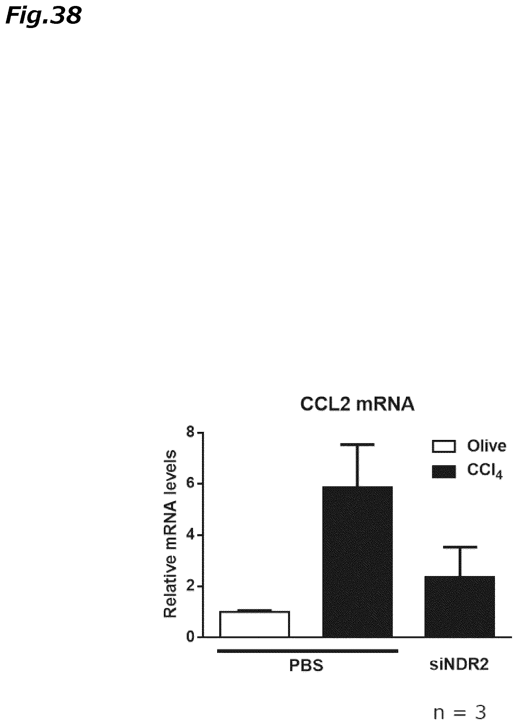

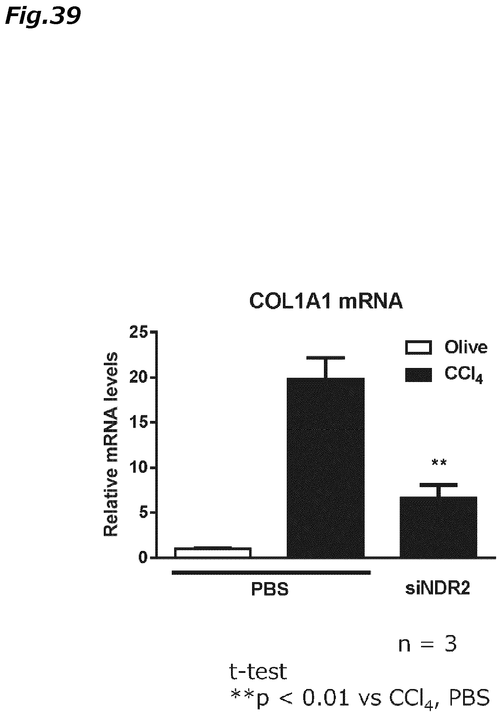

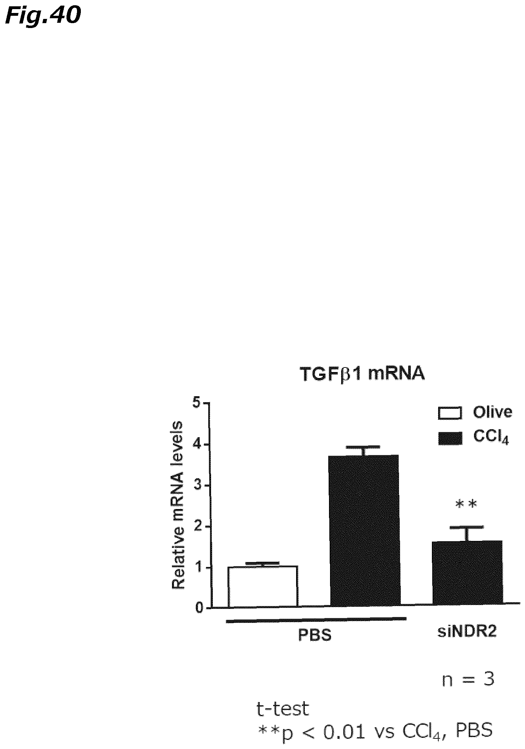

FIG. 34 illustrates the results of real-time PCR of NDR2 when Ambion In Vivo siRNA for NDR2 was administered via tail vein to the CCl₄ model mice. FIGS. 35 to 40 illustrate the results of real-time PCR of CYR61, CTGF, TNF, CCL2, COL1A1, and TGF-β1, which are fibrosis-associated genes, under the same conditions. By using the siRNAs of NDR2, the gene expression levels of profibrotic factors (CYR61 and CTGF), inflammatory markers (TNF and CCL2), and fibrosis markers (COL1A1 and TGF-β1) increased by CCl₄ were reduced. Therefore, it was suggested that NDR2 knockdown tended to improve fibrosis in the liver of the CCl₄ model mice.

### [Example 21: Analysis of Fibrosis Marker (αSMA) in CCl₄ Model]

In order to confirm that NDR2 knockdown is effective for fibrosis, NDR2 siRNAs were intravenously administered to the CCl₄ model mice to analyze the effect on fibrosis marker proteins.

To 8-weeks-old male C57BL/6J mice (Charles River Laboratories Japan, Inc.), an olive oil solution containing 12.5 v/v% CCl₄ (FUJIFILM Wako Pure Chemical Corporation) was intraperitoneally administered at 10 mL/kg by body weight on day 3 and 6 when the day on which the nucleic acid was first administered was day 0, thereby creating liver fibrosis model mice. Grouping was performed with body weight on the day before the initial administration of NDR2 siRNAs, and the design of the groups was as a PBS administration group not receiving CCl₄ (n = 3), a PBS administration group receiving CCl₄ (n =3), and a nucleic acid administration group receiving CCl₄ (n = 3). Using Ambion In Vivo siRNA (Thermo Fisher Scientific, Inc.) as NDR2 siRNA and Ambion In Vivo Negative control siRNA (Thermo Fisher Scientific, Inc.) as control siRNA, 0.2 mg/mL of a nucleic acid administration solution was prepared according to the product protocol of invivofectamine 3.0 using invivofectamine 3.0 Reagent (Thermo Fisher Scientific, Inc.). A nucleic acid administration solution containing Ambion In Vivo siRNA or Ambion In Vivo Negative control siRNA was administered via tail vein to the nucleic acid administration group so as to have a body weight of 2 mg/kg, and PBS (Thermo Fisher Scientific, Inc.) in an amount equivalent to that in the nucleic acid administration group was administered via tail vein to the solvent administration group on day 0 and 4.

A liver collected on day 8 was frozen and then crushed using a freeze-crush SK mill. A dissolution buffer (150 mM NaCl, 1% NP40, 0.1% SDS, 50 mM Tris-HCl pH 7.5, 1 mM EDTA, 1 mM Benzylsulfonyl fluoride, 2% protease inhibitor cocktail, 1% phosphatase inhibitor cocktail) was added to the liver crushed product, and an organ extract was prepared using a handy ultrasonic generator. β-Mercaptoethanol and Bromophenol blue were added to the supernatant obtained by centrifuging the organ extract at final concentrations of 5% and 0.025%, respectively. Thereafter, heating was performed at 95°C for 4 minutes to obtain a sample. SDS-PAGE was performed using the sample thus obtained, and proteins contained in the sample were separated by size. Thereafter, the separated protein was transferred to a PVDF membrane, and Western blotting was performed with an anti-αSMA antibody (Sigma-Aldrich Co. LLC.) and an anti-Vinculin antibody (Sigma-Aldrich Co. LLC.).

As the sequence of siRNA, Ambion In Vivo siRNA (SEQ ID NOs: 28 and 35) was used.

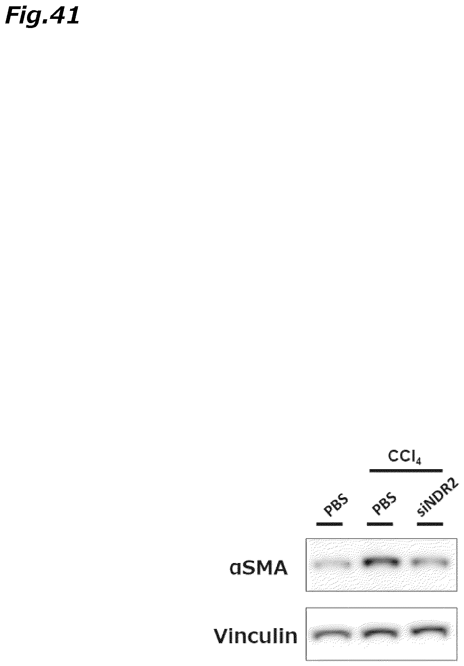

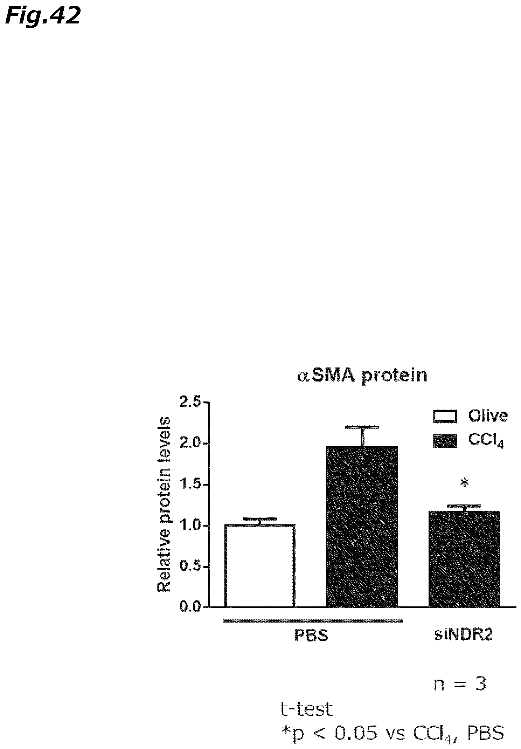

FIG. 41 illustrates the results of Western blotting of αSMA protein when NDR2 was knocked down. In addition, the value of the αSMA protein corrected by Vinculin is graphed in FIG. 42. The use of siRNAs of NDR2 reduced the amount of αSMA protein increased by CCl₄. Therefore, it was shown that αSMA protein in the liver of the CCl₄ model was suppressed by NDR2 knockdown.

Sequence **Listing**

## Claims

1. A therapeutic agent for fibrosis, comprising a nucleic acid suppressing expression of NDR2 gene as an active ingredient.

2. The therapeutic agent according to claim 1, wherein the fibrosis is pulmonary fibrosis, hepatic fibrosis, or kidney fibrosis.

3. The therapeutic agent according to claim 1 or 2, wherein the nucleic acid suppressing the expression of the NDR2 gene is an antisense nucleic acid.

4. The therapeutic agent according to claim 1 or 2, wherein the nucleic acid suppressing the expression of the NDR2 gene is an siRNA.

5. A method for treating fibrosis, comprising administering a nucleic acid suppressing expression of NDR2 gene to a subject.

6. A nucleic acid suppressing expression of NDR2 gene for use in the treatment of fibrosis.

7. Use of a nucleic acid suppressing expression of NDR2 gene in the manufacture of a therapeutic agent for fibrosis.

8. The method, the nucleic acid, or the use according to any one of claims 5 to 7, wherein the fibrosis is pulmonary fibrosis, hepatic fibrosis, or kidney fibrosis.
